# EUROPÄISCHE PATENTSCHRIFT

(11) **EP 1 436 606 B1**
(45) Veröffentlichungstag und Bekanntmachung des Hinweises auf die Patenterteilung: **27.10.2010**
(21) Anmeldenummer: 02802268.9
(22) Anmeldetag: 23.08.2002
(51) Int. Cl.: G01N 27/403, G01N 27/414

(54) **SENSOR-ANORDNUNG**
SENSOR ASSEMBLY
ENSEMBLE CAPTEUR

(30) Priorität: 16.10.2001 DE 10151021
(43) Veröffentlichungstag der Anmeldung: 14.07.2004
(73) Patentinhaber: Infineon Technologies AG, 85579 Neubiberg (DE)
(72) Erfinder: EVERSMANN, Björn-Oliver, 80336 München (DE); PAULUS, Christian, 82362 Weilheim (DE); STROMBERG, Guido, 80469 München (DE); THEWES, Roland, 82194 Gröbenzell (DE)
(74) Vertreter: Viering, Jentschura & Partner
(86) Internationale Anmeldenummer: PCT/DE2002/003098
(87) Internationale Veröffentlichungsnummer: WO 2003/038423

(56) Entgegenhaltungen:
- WO-A-00/62048
- WO-A-99/33018
- US-A- 5 902 044
- US-A- 5 965 452
- US-A- 6 154 580

## Beschreibung

Die Erfindung betrifft eine Sensor-Anordnung.

Aktuelle Entwicklungen in vielen Gebieten der Wissenschaft und Technik sind **dadurch gekennzeichnet, dass** ehemals voneinander unabhängige Bereiche zunehmend zusammengeführt werden. Ein Beispiel für einen interdisziplinären Bereich ist die Schnittstelle zwischen der Biologie und der Halbleitertechnik. Gegenstand aktueller Forschung ist beispielsweise die wirtschaftlich sehr interessante Kopplung zwischen biologischen Zellverbänden (wie beispielsweise Neuronen) und der Silizium-Mikroelektronik.

Gemäß einem Konzept wird ein biologisches System auf der Oberfläche eines halbleitertechnologischen Sensors aufgewachsen und dieses mittels auf der Oberfläche des Sensors matrixförmig angeordneter Sensor-Elektroden orts- bzw. zeitaufgelöst untersucht. Gemäß diesem Konzept können die Stoffwechselparameter der Zellen beispielsweise mittels Erfassens lokaler pH-Werte mit Hilfe von Ionensensitiven Feldeffekttransistoren (ISFETs) aufgenommen werden. Ein ISFET ist von seinem Grundprinzip her ähnlich wie ein Metall-Isolator-Halbleiter-Feldeffektransistor (MISFET) aufgebaut. Er unterscheidet sich von herkömmlichem MISFETs dadurch, dass die Leitfähigkeit des Kanalbereichs nicht mittels einer Metallelektrode, sondern mittels einer eine ionensensitive Schicht, einen Elektrolyten und eine Referenzelektrode aufweisenden Anordnung gesteuert wird. Mit anderen Worten steuern elektrisch geladene biologische Moleküle die Leitfähigkeit des ISFET, was als Sensorgröße erfasst wird.

Von besonderem Interesse ist das Untersuchen der Reaktion eines biologischen Systems auf eine elektrische Stimulation. Neuronen (Nervenzellen) können über Ionenkanäle in den Zellmembranen in bestimmten Bereichen ihrer Oberfläche einen kleinen elektrischen Strom erzeugen, der von einem darunterliegenden Sensor detektiert wird. Solche Impulse dauern typischerweise wenige Millisekunden, und die sich dadurch ausbildende elektrische Spannung liegt häufig unterhalb von 1mV. Um eine ausreichende Ortsauflösung zu erreichen, sollte der Abstand benachbarter Sensor-Elektroden zueinander in horizontaler bzw. vertikaler Richtung auf einer häufig matrixförmig angeordneten Sensoroberfläche, vorzugsweise weniger als 20µm sein, so dass die Oberfläche eines Sensors und die Querschnittsfläche einer Zelle ungefähr in der gleichen Größenordnung liegen. Diese Anforderungen sind mit der Silizium-Mikrotechnologie erreichbar.

Bei Sensor-Anordnungen mit einer ausreichend geringen Anzahl von Sensor-Feldern wird gemäß dem Stand der Technik das Ausgangssignal jedes Sensor-Feldes mit einer eigenen Leitung aus der Matrix herausgeführt und weiterverarbeitet. Bei einer größeren Anzahl von Sensor-Feldern oder kleiner werdenden Abständen benachbarter Sensor-Felder voneinander stößt dieses Prinzip wegen des hohen Platzbedarfs der hohen Anzahl von Leitungen an seine Grenzen.

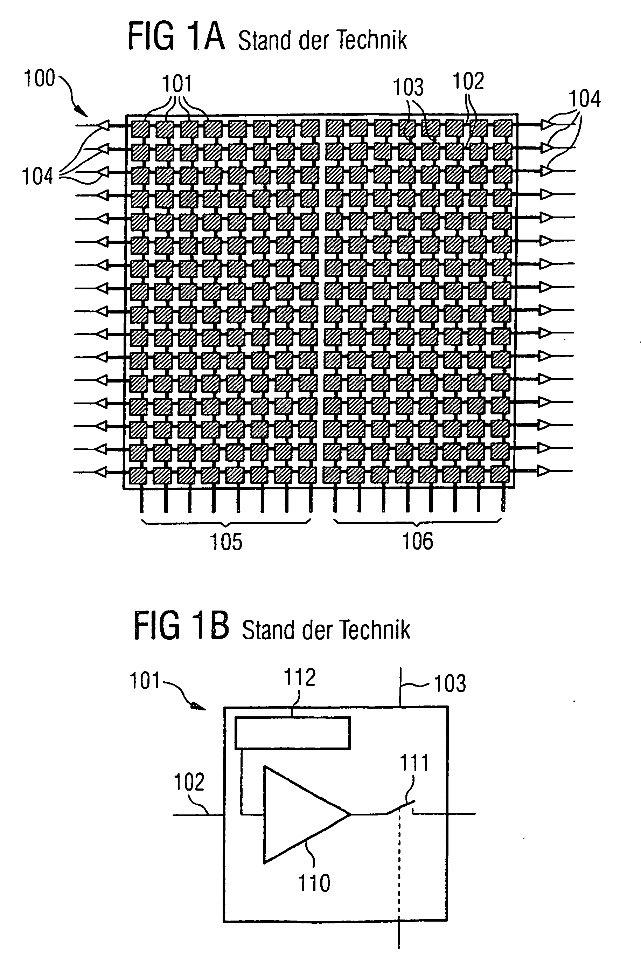

Bezugnehmend auf **Fig.1A, Fig.1B** wird im Weiteren ein aus dem Stand der Technik bekanntes Konzept beschrieben, mit dem es möglich ist, größere bzw. zunehmend dichte Anordnungen von Sensor-Elektroden auszulesen. In Fig.1A ist eine Sensor-Anordnung 100 mit einer Vielzahl von matrixförmig angeordneten Sensor-Elektroden 101 gezeigt. Die Sensor-Elektroden 101 sind (zumindest teilweise) mittels Zeilen-Leitungen 102 und Spalten-Leitungen 103 miteinander gekoppelt. In Randbereichen der Zeilen-Leitungen 102 ist jeweils eine elektrische Verstärker-Einrichtung 104 angeordnet. Wie ferner in Fig.1A gezeigt, ist die matrixförmige Sensor-Anordnung 100 in einen ersten Matrix-Bereich 105 und in einen zweiten Matrix-Bereich 106 aufgeteilt, die voneinander unabhängig betreibbar sind. Ähnlich wie bei dem Betrieb einer Speicher-Anordnung wird das Ausgangssignal einer bestimmten Sensor-Elektrode 101 über Schalter-Elemente 111 (vgl. Fig.1B) innerhalb der Sensor-Anordnung 100 auf eine gemeinsame Ausgangsleitung einer Zeile bzw. Spalte geschaltet. Die Grenzen der Leistungsfähigkeit des Systems ist gemäß dem in Fig.1A, Fig.1B gezeigten Konzept die auszulesende und zu verarbeitende Datenmenge. Soll eine Sensor-Anordnung mit einer ausreichend hohen Ortsauflösung (d.h. ausreichend vielen ausreichend dicht angeordneten Sensor-Elektroden) und mit einer ausreichend hohen Zeitauflösung (d.h. einer ausreichend hohen Auslesefrequenz) sowie mit einer ausreichend hohen Genauigkeit betrieben werden, so steigt die pro Zeit auszulesende Datenmenge auf Werte an, die derzeit nicht erreichbare Anforderungen an das technologisch zur Verfügung stehende Equipment stellen können. Die Signale an den Zeilen-Leitungen 102 und den Spalten-Leitungen 103 können wegen der immer noch sehr hohen Anzahl von Leitungen nicht parallel aus der Sensor-Anordnung 100 herausgeführt werden. Die Anforderungen an die hohe Datenmenge der n·m auszulesenden Sensor-Elektroden im Falle einer Matrix mit m Zeilen und n Spalten kann die Leistungsfähigkeit bekannter Technologien übersteigen.

In Fig.1B ist eine Sensor-Elektrode 101 im Detail dargestellt. Die Sensor-Elektrode 101 ist mit einer der Zeilen-Leitungen 102 und mit einer der Spalten-Leitungen 103 gekoppelt. Ist ein Schalter-Element 111 geschlossen, so ist die zugeordnete Sensor-Elektrode 101 ausgewählt und kann ausgelesen werden. Das von der Sensor-Fläche 112 in Form eines elektrischen Signals detektierte Sensor-Ereignis wird mittels eines Verstärker-Elements 110 verstärkt, bevor es über die Zeilen-Leitung 102 an den Rand der in Fig.1A dargestellten Sensor-Anordnung 100 übermittelt wird.

Zusammenfassend weisen aus dem Stand der Technik bekannte Sensor-Anordnungen zum ortsaufgelösten und zeitaufgelösten Erfassen analoger elektrischer Signale insbesondere den Nachteil auf, dass die n·m Sensor-Elektroden einzeln ausgelesen werden müssen und an einen signalverarbeitenden Schaltungsteil weitergeleitet werden müssen. Dadurch treten bei einer hohen Anzahl n·m von Sensor-Elektroden (m Zeilen, n Spalten) große, schnell zu verarbeitende Datenmengen auf, die mit ausreichender Genauigkeit verstärkt aus der Matrix herausgeleitet werden müssen. Dies übersteigt bei den zunehmenden Anforderungen an die Orts- und Zeitauflösung derartiger Systems die Leistungsgrenze bekannter Konzepte.

In [1] ist eine elektrisch ansteuerbare Sensoranordnung mit einer Vielzahl von Arrays offenbart, geeignet zur elektrochemischen Detektion molekularer Stoffe und geeignet zum Transport oder zur Handhabung geladener Moleküle. Ferner offenbart [1] eine aus Adressleitungen, Messleitungen und Biasleitungen bestehende Leitungsführung, die ein individuelles Auslesen von Sensorpositionen erlaubt. Die Leitungen sind teilweise in einer Zeilenrichtung und teilweise in einer Spaltenrichtung angeordnet. An den Sensorpositionen ist jeweils ein aus Mikroelektroden gebildetes Array angeordnet, welches von einem Multiplexer über elektronische Schaltelemente, die der jeweiligen Sensorposition zugeordnet sind, angesteuert werden kann.
[2] beschreibt ein gemultiplextes aktives biologisches Array mit einer Vielzahl adressierbarer Elektroden, welche in einer Mehrzahl von Zeilen und Spalten angeordnet sind. An jeder Elektrode ist ein Sample-and-hold-Schaltkreis angeschlossen, der durch Transistorschalter mit Zeilenleitungen und Spaltenleitungen verbunden werden kann. Ferner wird in [2] das Anbinden einer Matrix aktiver biologischer Elektroden an einen Zeilendekoder und einen Spaltendekoder beschrieben.
[3] offenbart einen integrierten Hotspot-Detektor. Im Einzelnen wird eine Matrix aus Thermosensoren bereitgestellt, von denen jeder über eine Spaltenleitung mit einem Spaltendekoder verbunden ist. Ferner ist jeder Thermosensor über einen zugehörigen FET-Schalter an einen Differenzschaltkreis angeschlossen, wobei der FET-Schalter über eine Zeilenleitung mit einem Zeilendekoder verbunden ist. Durch das Zusammenwirken von Zeilendekoder und Spaltendekoder wird ein lokaler Thermosensor ausgewählt und liefert dann einen Ausgangsstrom an den Differenzschaltkreis.
[4] beschreibt einen Fingerabdruck-Sensor mit einer Mehrzahl matrixförmig angeordneter Tastsensoren, von denen jeder über einen ersten Schalter-Transistor und eine Zeilenleitung mit einem gemeinsamen Ausgangsterminal verbunden ist. Ferner ist jeder Tastsensor über einen zweiten Schalter-Transistor und eine Spaltenleitung mit einer Spannungsquelle verbunden. Die Gates der Schalter-Transistoren werden von einem Scanner derart angesteuert, dass die Ausgabewerte der einzelnen Tastsensoren sequentiell ausgelesen werden können.

Der Erfindung liegt das Problem zugrunde, eine Sensor-Anordnung mit einer verbesserten Orts- und Zeitauflösung zu schaffen.

Das Problem wird durch eine Sensor-Anordnung mit den Merkmalen gemäß dem unabhängigen Patentanspruch gelöst.

Die erfindungsgemäße Sensor-Anordnung weist eine Mehrzahl von in einer ersten Richtung angeordneten Zeilen-Leitungen, eine Mehrzahl von in mindestens einer zweiten Richtung angeordneten Spalten-Leitungen und eine Mehrzahl von in Kreuzungsbereichen von Zeilen-Leitungen und Spalten-Leitungen angeordneten Sensor-Feldern auf. Jedes Sensor-Feld weist mindestens eine Kopplungs-Einrichtung zum elektrischen Koppeln von jeweils einer Zeilen-Leitung mit jeweils einer Spalten-Leitung und ein Sensor-Element auf, das der mindestens einen Kopplungs-Einrichtung zugeordnet ist, wobei das Sensor-Element derart eingerichtet ist, dass das Sensor-Element den elektrischen Stromfluss durch die mindestens eine zugeordnete Kopplungs-Einrichtung beeinflusst. Darüber hinaus weist die Sensor-Anordnung eine mit den Zeilen-Leitungen und den Spalten-Leitungen gekoppelte Dekodier-Einrichtung auf.

Die erfindungsgemäße Sensor-Anordnung ist **dadurch gekennzeichnet, dass** sie ferner ein an einem jeweiligen End-Abschnitt von mindestens einem Teil der Zeilen-Leitungen und von mindestens einem Teil der Spalten-Leitungen elektrisch gekoppeltes Mittel zum Erfassen eines jeweiligen Summen-Stromflusses aus den von den Sensor-Feldern der jeweiligen Leitung bereitgestellten elektrischen Einzel-Stromflüssen aufweist und dass die Dekodier-Einrichtung derart eingerichtet ist, dass sie mindestens einen Teil der elektrischen Summen-Stromflüsse, welche der Dekodier-Einrichtung über die Zeilen-Leitungen und die Spalten-Leitungen zuführbar sind, auswertet und basierend darauf entscheidet, an welchen Sensor-Elementen ein Sensor-Signal anliegen könnte.

Es sei betont, dass die Nomenklatur "Zeilen-Leitung" bzw. "Spalten-Leitung" keine orthogonale Matrix impliziert. Die in einer ersten Richtung verlaufenden Zeilen-Leitungen und die in mindestens einer zweiten Richtung verlaufenden Spalten-Leitungen können beliebige Winkel miteinander einschließen. Erfindungsgemäß können beliebig viele unterschiedliche Leitungen in beliebigen Winkeln über die Sensor-Anordnung gelegt werden und in Kreuzungsbereichen Kopplungs-Einrichtungen zwischengeschaltet werden, die einen bestimmten elektrischen Strom von einer Leitung in die andere Leitung "abzweigen". Eine der mindestens einen zweiten Richtung kann, muss aber nicht, orthogonal zu der ersten Richtung verlaufen. Die entlang der ersten Richtung angeordneten Zeilen-Leitungen sind insbesondere vorzugsweise zur Stromzuführung (aber auch zur Stromableitung), und die entlang der mindestens einen zweiten Richtung angeordneten Spalten-Leitungen sind insbesondere vorzugsweise zur Stromableitung vorgesehen.

Eine Grundidee der Erfindung beruht darauf, anders als gemäß dem Stand der Technik nicht jedes der n·m Matrixelemente einer Matrix mit m Zeilen und n Spalten einzeln auszulesen, sondern jeweils die Summensignale entlang einer Zeile bzw. entlang einer Spalte der Matrix auszulesen und über eine Korrelationsrechnung in einer Dekodier-Einrichtung die Zeilen- und Spalten-Signale derart miteinander zu verknüpfen bzw. rechnerisch auszuwerten, dass auf die Sensor-Signale der einzelnen Sensor-Elemente rückgeschlossen werden kann. Dadurch ist erreicht, dass statt n·m Signalen jedes Sensor-Feldes nur n+m Signale der Zeilen und Spalten auszulesen sind. Damit sind besonders bei hohen Werten n und m erhebliche numerische Vorteile verbunden und folglich höhere Ausleseraten erreichbar. Umgekehrt ist es bei einer festen Ausleserate möglich, die Dimensionen der einzelnen Sensor-Felder zu verkleinern und daher eine verbesserte Ortsauflösung zu erreichen.

Erfindungsgemäß wird die Tatsache ausgenützt, dass oft nur wenige Sensor-Elemente der Sensor-Anordnung ein Signal liefern. Auf den meisten Sensor-Elementen einer Sensor-Anordnung befindet sich beispielsweise bei einer Anwendung, welche die Untersuchung von auf der Oberfläche einer Sensor-Anordnung aufgewachsenen Neuronen zum Gegenstand hat, häufig lediglich Nährlösung oder solches Gewebe, das kein elektrisches Signal liefert. Dann weisen bei einem aktiven Sensor-Element die entsprechende Zeilen-Leitung und die entsprechende Spalten-Leitung ein korreliertes Signal auf, wobei auf Grund der Korrelation dasjenige Sensor-Element ermittelt werden kann, auf dem das Sensor-Ereignis stattgefunden hat. Detektiert zum Beispiel das in der k-ten Zeile und der 1-ten Spalte der Sensor-Anordnung angeordnete Sensor-Element ein Sensor-Ereignis, so wird im Wesentlichen zeitgleich in die k-te Zeilen-Leitung und in die 1-te Spalten-Leitung ein Einzelstromfluss eingespeist. Bei Auftreten von zeitlich bzw. intensitätsmäßig korrelierten Stromflüssen in der k-ten Zeilen-Leitung und der 1-ten Spalten-Leitung kann auf dasjenige Sensor-Element geschlossen werden, an dem das Sensor-Ereignis stattfand.

Während in bekannten Realisierungen von Sensor-Anordnungen alle Sensor-Felder nacheinander ausgelesen werden und deshalb n.m Signale in einem Zyklus ermittelt werden, werden in der erfindungsgemäßen Realisierung lediglich n+m Signale ausgegeben bzw. digitalisiert. Somit können wesentlich erhöhte Abtastraten, d.h. eine wesentlich verbesserte Zeitauflösung der Sensor-Anordnung erreicht werden.

Ein weiterer Vorteil besteht darin, dass eine echte Momentaufnahme der Potentialverhältnisse auf der aktiven Sensor-Oberfläche möglich ist. Während im konventionellen Fall die Matrixelemente nacheinander ausgelesen werden und somit zeitverschoben zueinander detektiert werden, kann im erfindungsgemäßen Fall die momentane Situation "festgehalten" und anschließend ausgewertet werden. Dies resultiert unter anderem aus der geringen Zahl auszulesender elektrischer Signale, die quasi instantan ausgelesen werden können.

Ferner weist die erfindungsgemäße Sensor-Anordnung den Vorteil auf, dass innerhalb der Sensor-Anordnung Schaltfunktionen zum Auswählen eines Sensor-Feldes entbehrlich sind. Dies ist gemäß dem Stand der Technik zum Auswählen eines bestimmten Sensor-Feldes erforderlich und hat eine hohe Störanfälligkeit aufgrund von kapazitiven Einkopplungen einer geschalteten Leitung auf andere Leitungen, beispielsweise Meßleitungen, zur Folge. Dadurch ist erfindungsgemäß die Nachweisempfindlichkeit erhöht. Ebenfalls unterdrückt sind erfindungsgemäß unerwünschte Wechselwirkungen eines Sensor-Feldes mit dem darauf angeordneten Untersuchungsobjekt (beispielsweise einem Neuron) infolge galvanischer, induktiver oder kapazitiver Einkopplungen.

Bevorzugte Weiterbildungen ergeben sich aus den abhängigen Ansprüchen.

Insbesondere kann die Dekodier-Einrichtung der erfindungsgemäßen Sensor-Anordnung in eine Zeilendekodier-Einrichtung, der die elektrischen Summen-Stromflüsse der Zeilen-Leitungen zuführbar sind, und in eine Spaltendekodier-Einrichtung, der die elektrischen Summen-Stromflüsse der Spalten-Leitungen zuführbar sind, aufgeteilt sein. Die Zeilendekodier-Einrichtung ist derart eingerichtet, dass aus mindestens einem Teil der elektrischen Summen-Stromflüsse der Zeilen-Leitungen unabhängig von den Summen-Stromflüssen der Spalten-Leitungen Informationen über diejenigen Sensor-Elemente ermittelbar sind, an denen möglicherweise ein Sensor-Signal anliegt. Die Spaltendekodier-Einrichtung ist derart eingerichtet, dass aus mindestens einem Teil der elektrischen Summen-Stromflüsse der Spalten-Leitungen unabhängig von den Summen-Stromflüssen der Zeilen-Leitungen Informationen über diejenigen Sensor-Elemente ermittelbar sind, an denen möglicherweise ein Sensor-Signal anliegt. Ferner ist die Dekodier-Einrichtung derart eingerichtet, dass mittels gemeinsamen Auswertens der von der Zeilendekodier-Einrichtung und der Spaltendekodier-Einrichtung ermittelten Informationen diejenigen Sensor-Elemente ermittelbar sind, an denen ein Sensor-Signal anliegt.

Indem anschaulich die Summen-Stromflüsse der Zeilen-Leitungen und der Spalten-Leitungen zunächst voneinander unabhängig dekodiert werden, ist die Geschwindigkeit der Dekodierung erhöht und mit geringerem Ressourcenaufwand möglich. Durch Auffinden simultaner Aktivierungsmuster der Sensor-Felder in Zeilen- bzw. Spalten-Leitungen kann durch Abgleich der Summen-Stromflüsse in den Zeilen- und Spalten-Leitungen die Position der aktiven Sensoren bestimmt werden. Auch ist es möglich, dass selbst die Summen-Stromflüsse unterschiedlicher Zeilen-Leitungen (oder unterschiedlicher Spalten-Leitungen) zunächst unabhängig von den Summen-Stromflüssen anderer Zeilen-Leitungen (oder anderer Spalten-Leitungen) ausgewertet und diese separaten Ergebnisse danach abgeglichen werden.

Ferner kann die Dekodier-Einrichtung derart eingerichtet sein, dass das Ermitteln von denjenigen Sensor-Elementen, an denen ein Sensor-Signal anliegt, erfolgt, indem eine Fourier-Transformation der zeitabhängigen Summen-Stromflüsse der Zeilen-Leitungen und der Spalten-Leitungen durchgeführt wird, die Fourier-transformierten Summen-Stromflüsse der Zeilen-Leitungen und der Spalten-Leitungen paarweise miteinander multipliziert werden und mit den paarweise miteinander multiplizierten Summen-Stromflüssen eine Fourier-Rücktransformation durchgeführt wird.

Mit anderen Worten werden bei der Korrelationsrechnung die zeitabhängigen Summen-Stromflüsse aller Zeilen- und Spalten-Leitungen einer Fourier-Transformation (vom Zeitraum in den Frequenzraum) unterzogen und im Frequenzraum paarweise miteinander multipliziert. Eine Fourier-Rücktransformation dieses Produktes der Summen-Stromflüsse in Zeilen- bzw. Spalten-Leitungen vom Frequenzraum in den Zeitraum liefert dann das ursprüngliche Signal im Zeitraum an denjenigen Sensor-Feldern, die am Kreuzungspunkt des jeweiligen Paars von Zeilen-Leitungen und Spalten-Leitungen liegt. Die Ergebnisse einer derartigen Auswertetechnik sind besonders gut, wenn nur ein geringer Anteil der im Wesentlichen matrixförmig angeordneten Sensor-Felder ein Signal liefert und je größer die Anzahl von Daten ist, die in der Korrelationsrechnung berücksichtigt werden. Mit anderen Worten wird anschaulich ein zweidimensionales Bild einer Potentialverteilung auf den Sensor-Feldern einer Sensor-Anordnung nicht wie gemäß dem Stand der Technik durch sukzessives Auslesen der einzelnen Matrixelemente, sondern durch eine Rückberechnung des Bildes aus den Summen-Stromflüssen von den Zeilen-Leitungen und Spalten-Leitungen der matrixförmigen Sensor-Anordnung ermittelt. Dadurch werden die günstigen Eigenschaften einer Fourier-Transformation erfindungsgemäß verwendet. Die Korrelationsrechnungen bewirken den weiteren Vorteil, dass Rauschen als unkorreliertes Signal bei der Korrelationsrechnung weitgehend eliminiert wird, so dass die erfindungsgemäße Auswertetechnik eine höhere Fehlerrobustheit und eine verbesserte Nachweisempfindlichkeit zur Folge hat. Es ist anzumerken, dass die zur Auswertung verwendete Transformation nicht notwendigerweise eine Fourier-Transformation sein muss. Allgemein kann eine beliebige geeignete Spektraltransformation (z.B. Laplace-Transformation, Diskrete Sinus Transformation [DSC], Diskrete Cosinus Transformation [DST]) verwendet werden.

Gemäß einer anderen Weiterbildung ist die Dekodier-Einrichtung derart eingerichtet, dass zum Ermitteln, ob an einem Sensor-Element ein Sensor-Signal anliegt, mindestens ein Summen-Stromfluss mindestens einer nebenliegenden Zeilen-Leitung und/oder mindestens einer nebenliegenden Spalten-Leitung verwendet wird.

"Nebenliegend" im Sinne der verwendeten Nomenklatur bedeutet nicht notwendigerweise direkt benachbart. Anschaulich werden beim Auswerten eines in einem Kreuzungsbereich einer Zeilen-Leitung und einer Spalten-Leitung angeordneten Sensor-Feldes nicht nur die Summen-Stromflüsse der beiden in diesem Kreuzungsbereich angeordneten Zeilen- und Spalten-Leitungen verwendet, sondern optional auch die Summen-Stromflüsse der an diese Zeilen-Leitung direkt oder durch mindestens eine Zeilen-Leitung getrennt angeordneten anderen Zeilen-Leitungen verwendet. Auch kann nicht nur die in dem Kreuzungsbereich angeordnete Spalten-Leitung verwendet werden, sondern auch die daran direkt angrenzenden weiteren Spalten-Leitungen oder Spalten-Leitungen, die in einem vorgegebenen Abstand von der betrachteten Spalten-Leitung angeordnet sind. Zum Ermitteln des Sensor-Signals eines bestimmten Sensor-Feldes mit den Indizes ij in der matrixförmigen Sensor-Anordnung werden nicht nur Summen-Stromflüsse der betreffenden Spalte j und Zeile i verwendet, sondern werden zudem Informationen aus anderen Spalten l≠j und Zeilen k≠i verwendet. Dies ist besonders dann vorteilhaft, wenn ein erheblicher Anteil der Sensor-Felder der matrixförmigen Sensor-Anordnung zu einem bestimmten Zeitpunkt ein Signal aufweisen, d.h. wenn entlang einer Zeile oder entlang einer Spalte mehrere Sensor-Elemente gleichzeitig ein Sensor-Signal aufweisen. Aufgrund der kreuzweisen Überlagerung der einzelnen, sich zeitlich und räumlich überlagernden Sensor-Signale besteht dann nicht nur eine Abhängigkeit zwischen genau einem Zeilen- und einem Spaltensignal (wie bisher angenommen), sondern diese Abhängigkeiten erstrecken sich dann über eine Gruppe aktiver Zeilen- und Spalten-Leitungen. Diese Interdependenzen werden gemäß dieser Ausgestaltung berücksichtigt, um eine weiter verbesserte Erkennung und Lokalisierung der aktiven Sensor-Felder durchführen zu können. Gemäß diesem Konzept ist eine größere Toleranz gegenüber Rauschen und Parameterschwankungen der Sensoren sowie gegenüber der gleichzeitigen Aktivität mehrerer Sensoren erreicht. Einzige Voraussetzung ist, dass die Signale wenigstens geringfügig gegeneinander verschoben sind oder sich in ihren spektralen Beiträgen unterscheiden.

Vorzugsweise ist die Dekodier-Einrichtung derart eingerichtet, dass beim Ermitteln, ob an einem Sensor-Element ein Sensor-Signal anliegt, mindestens ein vorgegebenes zeitliches und/oder räumliches Referenzsignal verwendet wird. Die Dekodier-Einrichtung der erfindungsgemäßen Sensor-Anordnung kann ferner derart eingerichtet sein, dass zum Ermitteln, ob an einem Sensor-Element ein Sensor-Signal anliegt, das mindestens eine vorgegebene zeitliche und/oder räumliche Referenzsignal an das erfasste Signals angepasst wird.

Beispielsweise sendet ein Neuron (Nervenzelle) nach einer Stimulation mit einem elektrischen Signal ein zu erfassendes elektrisches Signal mit einer für diesen Vorgang charakteristischen Form aus. Verwendet man die Kenntnis über die zu erwartende zeitliche und/oder räumliche Form des zu erfassenden Signals, so kann diese Kenntnis verwendet werden, um entlang einzelner Zeilen oder Spalten überlagerte Signale unterschiedlicher Sensor-Felder voneinander zu trennen. Die Kenntnis von Signalverläufen, die als zeitliches oder räumliches Referenzsignal in der Dekodier-Einrichtung enthalten sein können, sind für viele andere biologische oder physikalisch-technische Systeme bekannt. Dadurch ist bei einer möglichen Überlappung von Signalen, d.h. bei gleichzeitiger Aktivität mehrerer Sensoren entlang einer Zeilen- oder Spalten-Leitung ein weiteres Konzept geschaffen, um diese Signale voneinander zu trennen.

Vorzugsweise ist die Dekodier-Einrichtung der erfindungsgemäßen Sensor-Anordnung derart eingerichtet, dass zum Ermitteln, ob an einem Sensor-Element ein Sensor-Signal anliegt, mindestens zwei zeitliche und/oder räumliche Referenzsignale an das erfasste Signal angepasst werden.

Das Ausnützen von Vorwissen über den wahrscheinlichen Verlauf der Signale kann dahingehend ergänzt werden, dass auch Überlagerungen von Referenzsignalen während des Detektionsprozesses berücksichtigt werden. Mit anderen Worten kann das Anpassen eines vorgegebenen zeitlichen und/oder räumlichen Referenzsignals an ein erfasstes Signal auch unter Verwendung einer Mehrzahl von Referenzsignalen erfolgen. In diesem Falle bezieht die Dekodier-Einrichtung auch die Tatsache in die Auswertung der von den Stromsignalen der Zeilen- und Spalten-Leitung mit ein, dass an einem Sensor-Feld gleichzeitig mehrere Sensor-Signale anliegen.

Die Sensor-Anordnung der Erfindung kann ferner derart eingerichtet sein, dass zum Ermitteln, ob an einem Sensor-Signal zu einem zweiten Zeitpunkt ein Sensor-Signal anliegt, eine vorgegebene Referenzinformation über Sensor-Signale zu einem ersten Zeitpunkt verwendet wird, welcher erste Zeitpunkt zeitlich vor dem zweiten Zeitpunkt liegt.

Insbesondere kann die Dekodier-Einrichtung als Maximum-Likelihood Sequence Estimation-Dekoder oder als Maximum a posteriori-Dekoder ausgestaltet sein.

Das Ermitteln von denjenigen Sensor-Feldern, an denen ein Sensor-Signal anliegt, wird gemäß der beschriebenen Weiterbildung unter Verwendung von vorbekannten Informationen über den wahrscheinlichen Verlauf der Signale, d.h. auf Basis bekannter Referenzinformationen durchgeführt. Dies kann unter Verwendung von aus dem Stand der Technik bekannten statistischen Verfahren wie beispielsweise dem Maximum-Likelihood Sequence Estimation-Verfahren, dem Maximum a Posteriori-Verfahren oder anderer hierfür geeigneter Verfahren erfolgen. Das Maximum-Likelihood Sequence Estimation-Verfahren stellt allgemein ein Schätzverfahren bei einer gegebenen Statistik zur Bestimmung von Parametern aus einer gegebenen Stichprobe einer Zufallsgröße dar, wobei gemäß diesem Verfahren die Werte der Parameter derart ermittelt werden, dass eine sogenannte "Likelihood-Funktion" ein Maximum annimmt. Anschaulich wird bei dem Maximum-Likelihood Sequence Estimation-Verfahren die wahrscheinlichste Fortentwicklung eines Systems auf der Basis von Anfangs- bzw. Randbedingungen ermittelt. In einem sogenannten Trellis-Diagramm, in dem unterschiedliche mögliche Zustände des Systems zu unterschiedlichen Zeitpunkten dargestellt sind, wird derjenige Pfad von Systemzuständen ermittelt, der mit einer erfassten Sequenz die beste Übereinstimmung aufweist. Methoden wie das Maximum Likelihood Sequence Estimation-Verfahren oder das Maximum a posteriori-Verfahren können dazu beitragen, aus einer gegebenen Sequenz von Abtastwerten eine Schätzung für eine Dekomposition in unterschiedliche Mustersignale durchzuführen. Unter Verwendung der beschriebenen Verfahren ist es möglich, überlagerte Mustersignale voneinander zu trennen, wenn sie nur mindestens ein Abtastintervall gegeneinander verschoben sind. Im Falle der erfindungsgemäßen Sensor-Anordnung ist es daher ausreichend, dass überlagerte Signale um mindestens ein Sensor-Feld gegeneinander verschoben sind. Insbesondere ist darauf hinzuweisen, dass die Mustersignale nicht exakt von Anfang an bekannt sein müssen. Es ist eine Adaption, d.h. ein Anpassen, der Mustersignale an die tatsächlich generierten Signale möglich. Mit den beschriebenen Verfahren ist es möglich, Einzelsignale aus einem Sensor-Signal, das aus überlagerten Einzelsignalen gebildet ist, zu rekonstruieren.

Gemäß einer weiteren Ausgestaltung der erfindungsgemäßen Sensor-Anordnung kann diese eine Spannungsquelle aufweisen, die derart mit zumindest einem Teil der Zeilen-Leitungen und der Spalten-Leitungen gekoppelt ist, dass zumindest einem Teil der Kopplungs-Einrichtungen eine vorgegebene Potentialdifferenz bereitgestellt ist.

Beispielsweise kann an zumindest einem Teil der Spalten-Leitungen ein erstes Referenzpotential (beispielsweise eine Versorgungsspannung V_{dd}) angelegt werden und zumindest ein Teil der Zeilen-Leitungen auf ein zweites Referenzpotential (beispielsweise ein unteres Bezugpotential Vₛₛ wie das Massepotential) gelegt werden. Liegt an jeder der Kopplungs-Einrichtungen in Kreuzungsbereichen der Zeilen- bzw. Spalten-Leitungen, an welche die beschriebenen Referenzpotentiale angelegt sind, die gleiche elektrische Spannung, so fließt durch jede Kopplungs-Einrichtung der gleiche Ruhestrom. Ein Sensor-Ereignis moduliert die Spannung am Kopplungs-Element und somit den Stromfluss, der damit ein direktes Maß für die Sensor-Ereignisse an dem mit der jeweiligen Kopplungs-Einrichtung gekoppelten Sensor-Element darstellt.

Vorzugsweise ist mindestens eine Kopplungs-Einrichtung eine von dem zugehörigen Sensor-Element gesteuerte Stromquelle oder ein von dem zugehörigen Sensor-Element gesteuerter Widerstand.

Mit anderen Worten hängt der elektrische Stromfluss durch eine Kopplungs-Einrichtung bei einer Ausgestaltung der Kopplungs-Einrichtung als von dem zugehörigen Sensor-Element gesteuerte Stromquelle von dem Vorliegen bzw. Nichtvorliegen eines Sensor-Ereignisses an dem Sensor-Element ab. Auch kann der elektrische Widerstand der Kopplungs-Einrichtung in charakteristischer Weise davon abhängen, ob an dem zugeordneten Sensor-Element ein Sensor-Ereignis stattfindet oder nicht. Bei einem derart veränderbaren Widerstand ist der Stromfluss durch die Kopplungs-Einrichtung bei einer festen Spannung zwischen den zugeordneten Zeilen- und Spalten-Leitungen ein direktes Maß für die an dem Sensor-Element erfolgten Sensor-Ereignisse. Indem die Kopplungs-Einrichtung als von dem zugehörigen Sensor-Element gesteuerte Stromquelle oder von dem zugehörigen Sensor-Element gesteuerter Widerstand ausgebildet ist, ist eine wenig aufwändige Realisierung der Kopplungs-Einrichtungen ermöglicht.

Vorzugsweise weist mindestens eine Kopplungs-Einrichtung einen Detektions-Transistor mit einem mit einer der Zeilen-Leitungen gekoppelten ersten Source-/Drain-Anschluss, mit einem mit einer der Spalten-Leitung gekoppelten zweiten Source-/Drain-Anschluss und mit einem mit dem der Kopplungs-Einrichtung zugeordneten Sensor-Element gekoppelten Gate-Anschluss auf.

Anschaulich wird die Leitfähigkeit des Gate-Bereichs des Detektions-Transistors, vorzugsweise ein MOS-Transistor, dadurch beeinflusst, ob an dem zugeordneten Sensor-Element ein Sensor-Ereignis stattfindet oder nicht. Ist dies der Fall, d.h. werden beispielsweise von einem Neuron auf dem Sensor-Element über Ionenkanäle elektrisch geladene Partikel (beispielsweise Natrium- und Kaliumionen) in unmittelbare Nähe zum Sensor-Element gebracht, so verändern diese elektrisch geladenen Partikel die Ladungsmenge auf dem Gate-Anschluss des Detektions-Transistors, wodurch die elektrische Leitfähigkeit des Kanalbereichs zwischen den beiden Source-/Drain-Anschlüssen des Detektions-Transistors charakteristisch beeinflusst wird. Dadurch wird der Stromfluss durch die Kopplungs-Einrichtung charakteristisch beeinflusst, so dass die jeweilige Kopplungs-Einrichtung einen veränderten Beitrag zu dem Summen-Stromfluss der jeweiligen Zeilen- bzw. Spalten-Leitung liefert. Die Ausgestaltung der Kopplungs-Einrichtung als Detektions-Transistor stellt eine wenig aufwändige und platzsparende Realisierung dar, welche eine kostengünstige Herstellung und eine hohe Integrationsdichte von Sensor-Feldern ermöglicht. Durch die einfache schaltungstechnische Realisierung der Sensor-Felder der erfindungsgemäßen Sensor-Anordnung können die Zellen sehr klein realisiert werden, was eine hohe räumliche Auflösung des Sensors erlaubt.

Ferner kann mindestens eine Kopplungs-Einrichtung der erfindungsgemäßen Sensor-Anordnung eine Kalibrier-Einrichtung zum Kalibrieren der Kopplungs-Einrichtung aufweisen.

Bei den halbleitertechnologischen Bauelementen eines Sensor-Feldes handelt es sich in der Regel um integrierte Bauelemente, wie beispielsweise MOS-Transistoren. Da diese integrierten Bauelemente innerhalb eines Sensor-Feldes üblicherweise sehr klein ausgebildet werden, um eine große Ortsauflösung zu erreichen, tritt eine statistische Streuung ihrer elektrischen Parameter (beispielsweise Schwellenspannungen beim MOSFET) aufgrund von Schwankungen in der Prozessführung beim Herstellungsverfahren auf.

Die Abweichung der Schwellenspannungen und anderer Parameter kann beispielsweise kompensiert werden, indem eine Kalibrierung beispielsweise mit Hilfe einer Datentabelle vorgenommen wird. Hierfür wird an einzelne Sensor-Felder der matrixförmigen Sensor-Anordnung jeweils ein elektronisches Referenzsignal angelegt, und die gemessenen Stromstärken der entsprechenden Sensor-Elemente etwa in einer Tabelle abgelegt. Im Messbetrieb dient diese Tabelle, die als Datenbank in die Dekodier-Einrichtung integriert werden kann, zur Umrechnung möglicherweise fehlerbehafteter Messwerte. Dies entspricht einer Kalibrierung.

Alternativ kann die Kalibrier-Einrichtung der erfindungsgemäßen Sensor-Anordnung einen Kalibrier-Transistor mit einem mit der Zeilen-Leitung gekoppelten ersten Source-/Drain-Anschluss, mit einem mit dem Gate-Anschluss des Detektions-Transistors sowie mit einem mit dem zugeordneten Sensor-Element gekoppelten Kondensator gekoppelten zweiten Source-/Drain-Anschluss und mit einem mit einer weiteren Spalten-Leitung gekoppelten Gate-Anschluss aufweisen, wobei mittels der weiteren Spalten-Leitung an den Gate-Anschluss des Kalibrier-Transistors eine elektrische Kalibrier-Spannung anlegbar ist.

Gemäß der beschriebenen Verschaltung, bei der verglichen mit der oben beschriebenen einfachen Ausgestaltung der Kopplungs-Einrichtung als Detektions-Transistor ein weiterer Transistor, nämlich der Kalibrier-Transistor und ein Kondensator erforderlich sind, kann die Abweichung eines Parameters, wie beispielsweise der Schwellenspannung des Detektions-Transistors kompensiert werden, indem an die weitere Spalten-Leitung ein elektrisches Potential angelegt wird, infolgedessen der Kalibrier-Transistor leitet und ein Knoten zwischen dem Kondensator und dem Gate-Anschluss des Detektions-Transistors auf ein elektrisches Kalibrierungspotential aufgeladen wird. Dieses Kalibrierungspotential ergibt sich aus einem in die Zeilen-Leitung eingeprägten elektrischen Strom, der durch den als Diode wirkenden Detektions-Transistor in die Spalten-Leitung abfließt. Wird der Kalibrier-Transistor wieder nichtleitend, weil die an die weitere Spalten-Leitung angelegte Spannung abgeschaltet wird, verbleibt auf dem Gate-Anschluss des Detektions-Transistors ein elektrisches Potential, das für jedes Sensor-Feld der Sensor-Anordnung eine Korrektur der Schwellenspannung des jeweiligen Detektions-Transistors zulässt. Daher ist die Fehlerrobustheit der erfindungsgemäßen Sensor-Anordnung bei Verwendung einer Kalibrier-Einrichtung mit einem Kalibrier-Transistor und einem Kondensator verbessert. Insbesondere kann mittels Einprägens eines Nullstromes eine beliebige Kopplungs-Einrichtung auch deaktiviert werden. Ist der Kalibrier-Transistor leitend und wird in die Zeilen-Leitung kein Strom (Nullstrom) eingeprägt, so wird das Potential am Gate-Anschluss des Detektions-Transistors soweit reduziert, dass der Detektions-Transistor nichtleitend wird und nach Abschalten des Kalibrier-Transistors entsprechend deaktiviert bleibt. Dies bedeutet, dass das zugehörige Sensor-Feld unabhängig vom Signal des angeschlossenen Sensor-Elements kein Signal zum Summensignal der Zeilen- und Spalten-Leitungen beiträgt. Insbesondere trägt dieses Sensor-Feld auch nicht zum Rauschsignal auf den betroffenen Zeilen- und Spalten-Leitungen bei, weshalb die spätere Analyse der Signale an den verbleibenden, noch aktiven Sensor-Feldern vereinfacht ist.

Die Rauschleistung aller aktivierten Sensor-Felder addiert sich auf den jeweiligen Zeilen- bzw. Spalten-Leitungen. Bei einer Anwendung der erfindungsgemäßen Sensor-Anordnung für neuronale Signale und den sich daraus ergebenden geometrischen und elektrischen Randbedingungen ist häufig die dominante Störgröße ein im Detektions-Transistor erzeugtes 1/f-Rauschsignal. Die Rauschleistung dieser Störgröße nimmt bei abnehmender aktiver Bauteilfläche zu. Sind an einer Zeilen- oder Spalten-Leitung nun eine Vielzahl von Sensor-Feldern angeschlossen, tragen alle zum Rauschsignal auf der Summenleitung bei. Mittels Deaktivierens einzelner Sensor-Felder unter Verwendung des zuvor beschriebenen Konzepts oder auch durch den Einsatz eines geeigneten Deaktivierungsschalters beispielsweise im Signalstrompfad des Sensor-Feldes, kann das Signal-Rausch-Verhältnis auf den Summenleitungen und somit die Analyse der Signale verbessert werden. Die Möglichkeit der Deaktivierung einzelner Sensor-Felder ist insbesondere deshalb vorteilhaft, da häufig nur wenige der Sensor-Elemente mit Neuronen belegt sind und somit potentiell ein Signal liefern können. Auch wenn ein Sensor-Element mit einem Neuron belegt ist, ist nicht sichergestellt, dass die elektrische Kopplung zwischen Zellmembran und Sensor-Oberfläche zur Übertragung eines Signals in die Kopplungs-Einrichtung ausreicht. Werden alle diese Sensor-Felder deaktiviert, kann hierdurch der Signal-Rausch-Abstand auf den Summenleitungen wesentlich verbessert werden.

Ferner kann mindestens eine Kopplungs-Einrichtung der erfindungsgemäßen Sensor-Anordnung ein Verstärker-Element zum Verstärken des elektrischen Einzel-Stromflusses der Kopplungs-Einrichtung aufweisen. Insbesondere kann das Verstärker-Element einen Bipolar-Transistor mit einem mit der Zeilen-Leitung gekoppelten Kollektor-Anschluss, einem mit der Spalten-Leitung gekoppelten Emitter-Anschluss und einem mit dem zweiten Source-/Drain-Anschluss des Detektions-Transistor gekoppelten Basis-Anschluss aufweisen.

Indem ein Bipolar-Transistor als Verstärker-Element verwendet wird, dessen Ausbildung mit herkömmlichen halbleitertechnologischen Methoden wenig aufwändig und daher kostengünstig möglich ist, ist ein leistungsstarkes Verstärker-Element einer geringen Dimension auf dem Sensor-Feld bereitgestellt, mit dem eine hohe Verstärkung der oft kleinen Stromflüsse erreicht werden können. Dadurch kann die Empfindlichkeit der Sensor-Anordnung erhöht werden.

Vorzugsweise hat zumindest ein Teil der Zeilen-Leitungen und der Spalten-Leitungen eine Verstärker-Einrichtung zum Verstärken des in der jeweiligen Zeilen-Leitung bzw. Spalten-Leitung fließenden elektrischen Summen-Stromflusses.

Zumindest ein Sensor-Element der Sensor-Anordnung kann ein Ionensensitiver Feldeffekttransistor (ISFET) sein.

Die Funktionalität eines ISFET ist oben beschrieben. Ein ISFET stellt ein Sensor-Element dar, das in einem standardisierten halbleitertechnologischen Verfahren mit geringem Aufwand herstellbar ist und das eine hohe Nachweisempfindlichkeit aufweist.

Auch kann zumindest ein Sensor-Element auf der Sensor-Anordnung ein auf elektromagnetische Strahlung empfindlicher Sensor sein.

Ein auf elektromagnetische Strahlung empfindlicher Sensor, beispielsweise eine Fotodiode oder ein anderes fotosensitives Element, ermöglicht den Betrieb der Sensor-Anordnung als optischen Sensor mit einer hohen Wiederholrate. Die erfindungsgemäße Sensor-Anordnung weist allgemein den Vorteil auf, dass an das Sensor-Element keine weiteren Anforderungen gestellt werden, außer dass ein Sensor-Ereignis ein elektrisches Signal hervorrufen soll.

Die Sensor-Felder der Sensor-Anordnung sind vorzugsweise im Wesentlichen rechteckförmig ausgebildet.

In diesem Fall sind die Sensor-Felder vorzugsweise matrixförmig angeordnet. Die Spalten- und Zeilen-Leitungen können orthogonal zueinander entlang der Kanten der rechteckigen Sensor-Felder ausgebildet werden. Mit anderen Worten können die Zeilen-Leitungen und die Spalten-Leitungen der erfindungsgemäßen Sensor-Anordnung miteinander einen im Wesentlichen rechten Winkel einschließen.

Gemäß einer alternativen Ausgestaltung der erfindungsgemäßen Sensor-Anordnung sind die Sensor-Felder im Wesentlichen wabenförmig ausgebildet. Als wabenförmig wird hier eine Ausgestaltung der Sensor-Felder bezeichnet, bei denen die Sensor-Felder sechseckig mit paarweise parallelen Seiten sind, weiter vorzugsweise mit 120° Winkeln an jeder Ecke des Sechsecks.

Im Falle einer wabenförmigen Ausgestaltung der Sensor-Felder können die Zeilen-Leitungen mit den Spalten-Leitungen einen Winkel von 60° einschließen, und unterschiedliche Spalten-Leitungen können zueinander entweder parallel sein oder miteinander einen Winkel von 60° einschließen.

Durch die Verwendung wabenförmiger Sensor-Felder wird eine besonders hohe Integrationsdichte von Sensor-Feldern erreicht, wodurch eine hohe räumliche Auflösung der Sensor-Anordnung erreicht ist.

Vorzugsweise ist die Sensor-Anordnung in mindestens zwei voneinander unabhängig betreibbare Bereiche aufgeteilt, wobei die Sensor-Anordnung derart eingerichtet ist, dass vorgebbar ist, welche der mindestens zwei Bereiche in einem bestimmten Betriebszustand betrieben werden. Die Bereiche können hierbei räumlich direkt benachbart angeordnet sein (z.B. Hälften, Quadranten) oder ineinander verschachtelt sein, beispielsweise derart, dass bei einer orthogonalen Anordnung von Sensor-Feldern die Kopplungs-Einrichtungen beispielsweise schachbrettartig an das eine oder das andere System von Spalten- und Zeilen-Leitungen angeschlossen sind.

Die matrixförmige Sensor-Anordnung kann also in unterschiedliche Segmente aufgeteilt sein (beispielsweise in vier Quadranten), um die Messgenauigkeit aufgrund verringerter Leitungskapazitäten zu erhöhen. Ist beispielsweise bekannt, dass in einem Bereich der Sensor-Anordnung Sensor-Ereignisse nicht auftreten können (beispielsweise weil in diesem Bereich keine Neuronen aufgewachsen sind), so muss nur der Restbereich der Sensor-Anordnung untersucht werden, auf dem Sensor-Ereignisse stattfinden können. Die Versorgung des nicht verwendeten Bereichs mit Versorgungsspannungen ist daher eingespart. Ferner sind Signale nur von demjenigen Bereich auszuwerten, in dem Sensor-Signale auftreten können. Auch kann es für bestimmte Anwendungen ausreichend sein, nur einen Teil-Bereich der Oberfläche der Sensor-Anordnung zu verwenden, der kleiner ist als die gesamte Oberfläche der Sensor-Anordnung. In diesem Fall kann der gewünschte Teil-Bereich zugeschaltet werden, was eine besonders schnelle und wenig aufwändige Ermittlung der Sensor-Ereignisse der auf dem Teil-Bereich angeordneten Sensor-Feldern ermöglicht.

Ausführungsbeispiele der Erfindung sind in den Figuren dargestellt und werden im Weiteren näher erläutert.

Es zeigen:
Figur 1A eine Sensor-Anordnung gemäß dem Stand der Technik,
Figur 1B eine Sensor-Elektrode der in Figur 1A gezeigten Sensor-Anordnung gemäß dem Stand der Technik,
Figur 2 eine Sensor-Anordnung gemäß einem ersten Ausführungsbeispiel der Erfindung,
Figur 3 eine Sensor-Anordnung gemäß einem zweiten Ausführungsbeispiel der Erfindung,
Figur 4A ein Sensor-Feld einer Sensor-Anordnung gemäß einem ersten Ausführungsbeispiel der Erfindung,
Figur 4B ein Sensor-Feld einer Sensor-Anordnung gemäß einem zweiten Ausführungsbeispiel der Erfindung,
Figur 5A ein Sensor-Feld einer Sensor-Anordnung gemäß einem dritten Ausführungsbeispiel der Erfindung,
Figur 5B ein Sensor-Feld einer Sensor-Anordnung gemäß einem vierten Ausführungsbeispiel der Erfindung,
Figur 5C ein Sensor-Feld einer Sensor-Anordnung gemäß einem fünften Ausführungsbeispiel der Erfindung,
Figur 5D ein Sensor-Feld einer Sensor-Anordnung gemäß einem sechsten Ausführungsbeispiel der Erfindung,
Figur 6 eine schematische Ansicht einer teilweise mit Neuronen bedeckten erfindungsgemäßen Sensor-Anordnung gemäß dem in Figur 3 gezeigten zweiten Ausführungsbeispiel der erfindungsgemäßen Sensor-Anordnung,
Figur 7 eine Sensor-Anordnung gemäß einem dritten Ausführungsbeispiel der Erfindung,
Figur 8 einen Maximum-Likelihood Sequence Estimation-Dekoder der in Figur 7 gezeigten Sensor-Anordnung gemäß einem ersten Ausführungsbeispiel der Erfindung,
Figur 9 ein Trellis-Diagramm,
Figur 10 eine Sensor-Anordnung gemäß einem vierten Ausführungsbeispiel der Erfindung,
Figur 11 einen Maximum-Likelihood Sequence Estimation-Dekoder der in Figur 10 gezeigten Sensor-Anordnung gemäß einem zweiten Ausführungsbeispiel der Erfindung.

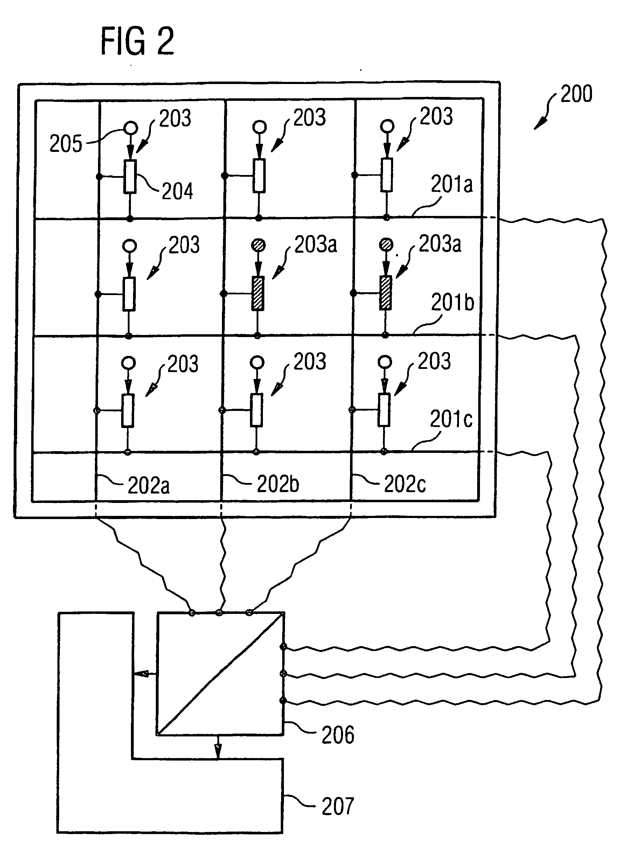

Im Weiteren wird bezugnehmend auf **Fig.2** eine Sensor-Anordnung gemäß einem ersten Ausführungsbeispiel der Erfindung beschrieben.

Die in Fig.2 gezeigte Sensor-Anordnung 200 weist drei in horizontaler Richtung angeordnete Zeilen-Leitungen 201a, 201b, 201c, drei in vertikaler Richtung angeordnete Spalten-Leitungen 202a, 202b, 202c und neun in den Kreuzungsbereichen der drei Zeilen-Leitungen 201a, 201b, 201c und Spalten-Leitungen 202a, 202b, 202c angeordnete Sensor-Feldern 203 mit einer Kopplungs-Einrichtung 204 zum elektrischen Koppeln von jeweils einer Zeilen-Leitung 201a, 201b oder 201c mit jeweils einer Spalten-Leitung 202a, 202b oder 202c und mit einem Sensor-Element 205, das der Kopplungs-Einrichtung 204 zugeordnet ist, wobei das Sensor-Element 205 derart eingerichtet ist, dass das Sensor-Element 205 den elektrischen Stromfluss durch die zugeordnete Kopplungs-Einrichtung 204 beeinflusst. Ferner weist die Sensor-Anordnung 200 ein an einem jeweiligen End-Abschnitt der Zeilen-Leitungen 201a, 201b, 201c und der Spalten-Leitungen 202a, 202b, 202c elektrisch gekoppeltes Mittel 206 zum Erfassen eines jeweiligen Summen-Stromflusses aus den von den Sensor-Feldern 203 der jeweiligen Zeilen- beziehungsweise Spalten-Leitungen bereitgestellten elektrischen Einzel-Stromflüssen. Die Sensor-Anordnung 200 hat ferner eine mit den Zeilen-Leitungen 201a, 201b, 201c und den Spalten-Leitungen 202a, 202b, 202c gekoppelte Dekodier-Einrichtung 207, die derart eingerichtet ist, das aus den elektrischen Summen-Stromflüssen, welche der Dekodier-Einrichtung 207 über die Zeilen-Leitungen 201a, 201b, 201c und die Spalten-Leitungen 202a, 202b, 202c zuführbar sind, die aktivierten Sensor-Elemente 203a ermittelbar sind, an denen ein Sensor-Signal anliegt.

In Fig.2 sind die beiden in den Kreuzungsbereichen der zweiten Zeile 201b und der zweiten und dritten Spalten 202b, 202c befindlichen aktivierten Sensor-Felder 203a optisch hervorgehoben.

Diese Sensor-Felder 203a sind solche, bei denen an dem Sensor-Element 205 ein Sensor-Ereignis erfolgt, infolgedessen das Sensor-Element 205 den Stromfluss durch die Kopplungs-Einrichtung 204 charakteristisch beeinflusst. Eine in Fig.2 nicht gezeigte Spannungsquelle stellt zwischen jeder der Zeilen-Leitungen 201a, 201b, 201c und jeder der Spalten-Leitungen 202a, 202b, 202c eine vorgegebene Potentialdifferenz bereit. Bei dieser festen Potentialdifferenz wird der Stromfluss durch die Kopplungs-Einrichtungen 204 der Sensor-Felder 203 durch die Sensor-Ereignisse an den zugeordneten Sensor-Elementen 205 charakteristisch beeinflusst. Anschaulich ist besonders an der zweiten Zeilen-Leitung 201b ein stark veränderter Stromfluss detektierbar, da zwei von drei Sensor-Feldern 203, mit denen die Zeilen-Leitung 201b gekoppelt ist, infolge eines Sensor-Ereignisses einen veränderten elektrischen Stromfluss aufweisen. Auch die zweite und dritte Spalten-Leitung 202b, 202c weisen einen (allerdings weniger stark) veränderten Stromfluss auf, da jeweils eines von drei mit diesen Spalten-Leitungen 202b, 202c gekoppelten Sensor-Feldern 203 einen veränderten Stromfluss aufweist. Die Summen-Stromflüsse entlang der Zeilen-Leitungen 201a bis 201c und der Spalten-Leitungen 202a bis 202c werden, wie schematisch in Fig.2 gezeigt, dem Mittel 206 zum Erfassen von Summen-Stromflüssen bereitgestellt, welches wiederum die erfassten Summen-Stromschlüsse der Dekodier-Einrichtung 207 bereitstellt. Es ist anschaulich verständlich, dass bei einer Untersuchung der Korrelation der Summenströme jeweils einer Zeilen-Leitung mit jeweils einer Spalten-Leitung ermittelbar ist, welche Sensor-Felder 203a aktiviert sind.

Die Dekodier-Einrichtung 207 der Sensor-Anordnung 200 ist derart eingerichtet, dass das Ermitteln von denjenigen Sensor-Elementen 205, an denen ein Sensor-Signal anliegt, erfolgt, indem eine Fourier-Transformation der zeitabhängigen Summen-Stromflüsse der Zeilen-Leitungen und der Spalten-Leitungen durchgeführt wird, die Fourier-transformierten Summen-Stromflüsse der Zeilen-Leitungen und der Spalten-Leitungen paarweise miteinander multipliziert werden und auf die paarweise miteinander multiplizierten Summen-Stromflüsse eine Fourier-Rücktransformation durchgeführt wird.

Anschaulich werden paarweise Kreuzkorrelationen von Zeilen-und Spalten-Leitungen berechnet. Die Ermittlung des Sensor-Signals eines speziellen Sensor-Elements 205 sᵢⱼ erfolgt mittels Berechnens paarweiser Kreuzkorrelationen der zeitabhängigen Summen-Stromflüsse aller Spalten j mit allen Zeilen i. Die Korrelationsrechnungen unter Verwendung der mathematischen Operation der Fourier-Transformation beziehungsweise der Fourier-Rücktransformation von dem Zeitraum in den Frequenzraum ermöglicht es, die zeitabhängigen Signale der einzelnen Sensor-Felder 203 zu bestimmen. Dies ist insbesondere deshalb möglich, da der elektrische Strom, der durch eine Kopplungs-Einrichtung 204 fließt, in der dazugehörigen Zeilen-Leitung sowie in der dazugehörigen Spalten-Leitung korreliert auftritt. Mit anderen Worten beeinflusst ein Sensor-Ereignis an einem Sensor-Element 205 eines aktivierten Sensor-Feldes 203a sowohl die damit gekoppelte Zeilen-Leitung als auch die damit gekoppelte Spalten-Leitung. Es ist eine vorteilhafte Eigenschaft der Fourier-Transformation, dass unkorrelierte Signale, das heißt Rauschsignale und dergleichen bei der Korrelationsrechnung zumindest zum Teil eliminiert werden. Mit Hilfe des beschriebenen Fourier-Transformationsverfahrens ist es daher möglich, aus den Summen-Stromsignalen der drei Zeilen-Leitungen 201a bis 201c und der Summen-Stromflüsse der drei Spalten-Leitungen 202a bis 202c, das heißt aus insgesamt 3+3=6 Summen-Stromsignalen, die aktivierten Sensor-Felder 203a zu ermitteln. Dagegen ist es bei aus dem Stand der Technik bekannten Verfahren erforderlich, separat die Signale der 3*3=9 Sensor-Felder 203 auszulesen und daher neun Signale zu erfassen. (Bei Vorrichtungen gemäß dem Stand der Technik können sowohl Ströme als auch Spannungen als informationstragende Größe aus der Sensor-Anordnung herausgeleitet werden.)Erfindungsgemäß ist daher die Anzahl der zu erfassenden Stromsignale reduziert, was eine erhöhte Geschwindigkeit der Auswertung und bei einer vorgegebenen maximalen Ausleserate eine Erhöhung der Anzahl der Sensor-Felder 203 einer Sensor-Anordnung 200 ermöglicht. Es ist darauf hinzuweisen, dass der numerische Vorteil um so höher ist, je höher die Anzahl der Zeilen- und Spalten-Leitungen ist. Im allgemeinen Fall reduziert sich die aus dem Stand der Technik erforderliche Bestimmung von n·m Stromsignalen bei m Spalten und n Zeilen zu m+n Summen-Stromsignalen, die erfindungsgemäß zu erfassen sind.

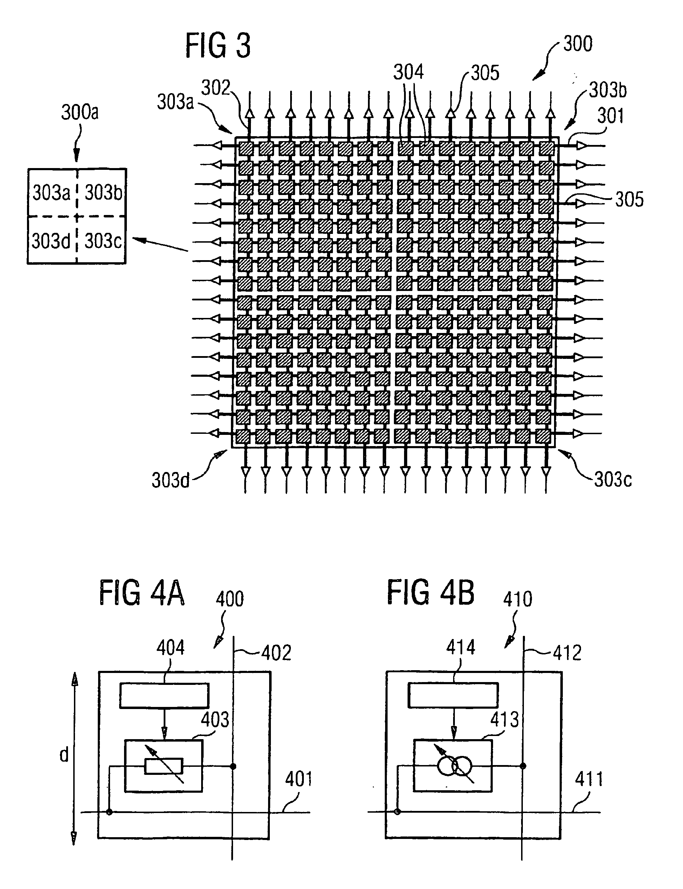

In **Fig.3** ist eine Sensor-Anordnung gemäß einem zweiten bevorzugten Ausführungsbeispiel der Erfindung gezeigt.

Die Sensor-Anordnung 300 ist ähnlich aufgebaut wie die bezugnehmend auf Fig.2 beschriebene Sensor-Anordnung 200. Insbesondere weist die Sensor-Anordnung 300 sechzehn Zeilen-Leitungen 301 und sechzehn Spalten-Leitungen 302 auf. Erfindungsgemäß sind daher 32 Summen-Stromsignale zu erfassen, wohingegen bei einem aus dem Stand der Technik bekannten Konzept 256 Stromsignale der 256 Sensor-Felder 304 zu erfassen wären. Bei der in Fig.3 gezeigten Sensor-Anordnung 300 sind die Sensor-Felder 304 rechteckförmig ausgebildet. Die Zeilen-Leitungen 301 und die Spalten-Leitungen 302 schließen miteinander einen rechten Winkel ein. Die Sensor-Anordnung 300 ist in vier voneinander unabhängig betreibbare Teil-Bereiche 303a, 303b, 303c, 303d aufgeteilt, wobei die Sensor-Anordnung 300 derart eingerichtet ist, dass vorgebbar ist, welche der vier Teil-Bereiche 303a bis 303d betrieben werden. Die Anordnung der vier Teil-Bereiche 303a bis 303d innerhalb der Sensor-Anordnung 300 ist in Fig.3 in der Prinzipskizze 300a gezeigt. Jede Zeilen-Leitung 301 und jede Spalten-Leitung 302 der Sensor-Anordnung 300 weist eine Verstärker-Einrichtung 305 zum Verstärken des in der jeweiligen Zeilen-Leitung 301 beziehungsweise Spalten-Leitung 302 fließenden elektrischen Summen-Stromflusses auf.

Möglichkeiten für den detaillierten Aufbau der Sensor-Felder 304 werden im Weiteren anhand bevorzugter Ausführungsbeispiele bezugnehmend auf Fig.4A bis Fig.5B erläutert.

**In** **Fig.4A** ist ein Sensor-Feld 400 gemäß einem ersten Ausführungsbeispiel der Erfindung gezeigt.

Das Sensor-Feld 400 ist in einem Kreuzungsbereich einer Zeilen-Leitung 401 und einer Spalten-Leitung 402 angeordnet. Über zwei elektrische Kreuzungspunkte ist die Zeilen-Leitung 401 mit der Spalten-Leitung 402 über eine Kopplungs-Einrichtung 403 gekoppelt. Die Kopplungs-Einrichtung 403 ist als von einem Sensor-Element 404 steuerbarer Widerstand ausgebildet. Mit anderen Worten führt ein Sensor-Ereignis an dem Sensor-Element 404 dazu, dass der elektrische Widerstand der Kopplungs-Einrichtung 403 in charakteristischer Weise beeinflusst wird. Das Sensor-Feld 400 ist ein Quadrat mit einer Seitenlänge d. Um eine für neurobiologische Zwecke ausreichend hohe Integrationsdichte von Sensor-Feldern 400 in einer Sensor-Anordnung zu erreichen, wird die Kantenlänge d des quadratischen Sensor-Feldes 400 vorzugsweise entsprechend klein gewählt.

In **Fig.4B** ist ein Sensor-Feld 410 gemäß einem zweiten Ausführungsbeispiel der Erfindung gezeigt.

Das Sensor-Feld 410 ist in einem Kreuzungsbereich einer Zeilen-Leitung 411 und einer Spalten-Leitung 412 angeordnet. Das Sensor-Feld 410 weist eine Kopplungs-Einrichtung 413 auf, mittels der über zwei elektrische Kopplungspunkte die Zeilen-Leitung 411 mit der Spalten-Leitung 412 gekoppelt ist. Gemäß dem in Fig.4B gezeigten Ausführungsbeispiel ist die Kopplungs-Einrichtung 413 als von dem Sensor-Element 414 gesteuerte Stromquelle ausgebildet. Mit anderen Worten führt ein Sensor-Ereignis an dem Sensor-Element 414 dazu, dass der elektrische Strom der gesteuerten Stromquelle 413 in charakteristischer Weise beeinflusst wird.

Als Kopplungs-Einrichtung 403 beziehungsweise 413 innerhalb eines Sensor-Feldes 400 beziehungsweise 410 ist also ein gesteuerter Widerstand oder eine gesteuerte Stromquelle mit einer linearen oder nichtlinearen Kennlinie bereitgestellt. Wesentlich ist, dass mit Hilfe einer geeigneten Verschaltung ein Stromfluss von einer Zeilen-Leitung in eine Spalten-Leitung verzweigt wird, welcher Stromfluss von einem Sensor-Ereignis charakteristisch beeinflusst wird.

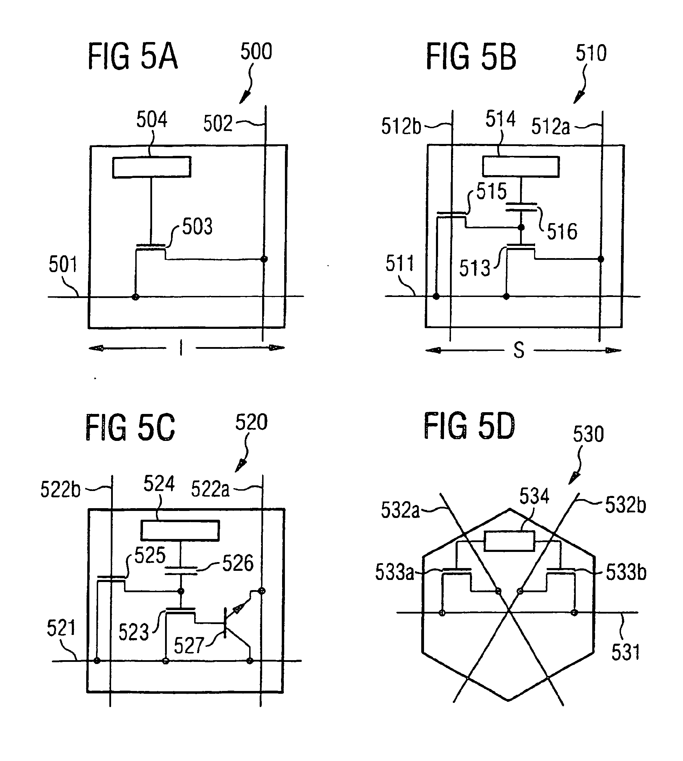

In **Fig.5A** ist ein Sensor-Feld 500 gemäß einem dritten Ausführungsbeispiel der Erfindung gezeigt.

Das in Fig.5A gezeigte Sensor-Feld 500 ist in einem Kreuzungsbereich einer Zeilen-Leitung 501 und einer Spalten-Leitung 502 angeordnet. Mittels einer als Detektions-Transistor 503 ausgebildeten Kopplungs-Einrichtung ist über zwei elektrische Kreuzungspunkte die Zeilen-Leitung 501 mit der Spalten-Leitung 502 gekoppelt. Der Detektions-Transistor 503 weist einen mit der Zeilen-Leitung 501 gekoppelten ersten Source-/Drain-Anschluss, einen mit der Spalten-Leitung 502 gekoppelten zweiten Source-/Drain-Anschluss und einen mit dem Sensor-Element 504 gekoppelten Gate-Anschluss auf. Die Länge 1 einer Seite des quadratisch ausgebildeten Sensor-Felds 500 ist vorzugsweise entsprechend klein, um eine ausreichend hohe Ortsauflösung zu erreichen.

Zwischen die Zeilen-Leitung 501 und die Spalten-Leitung 502 ist eine konstante elektrische Spannung angelegt. Erfolgt an dem Sensor-Element 504 ein Sensor-Ereignis, bei dem elektrisch geladene Partikel das Potential des Gate-Anschlusses des Detektions-Transistors 503 charakteristisch beeinflussen, so wird infolge des Sensor-Ereignisses die Leitfähigkeit des leitenden Kanals zwischen den beiden Source-/Drain-Anschlüssen des Detektions-Transistors 503 beeinflusst. Daher ist der elektrische Stromfluss zwischen dem ersten und dem zweiten Source-/Drain-Bereich des Detektions-Transistors 503 ein Maß für das an dem Sensor-Element 504 erfolgte Sensor-Ereignis. Mit anderen Worten ist das Sensor-Element 504 vor einem Sensor-Ereignis durch eine geeignete Maßnahme auf ein vorgegebenes elektrisches Potential gebracht, so dass zwischen den beiden Source-/ Drain-Anschlüssen des Detektions-Transistors 503 ein elektrischer Ruhestrom aus der Spalten-Leitung 502 in die Zeilen-Leitung 501 fließt. Wie das elektrische Potential des Gate-Anschlusses beeinflusst, beispielsweise weil ein mit dem Sensor-Element 504 gekoppeltes Neuron einen elektrischen Puls abgibt, so wird infolge der veränderten elektrischen Leitfähigkeit des Detektions-Transistors 503 der Querstrom zwischen der Zeilen-Leitung 501 und der Spalten-Leitung 502 verändert.

Bezugnehmend auf **Fig.5B** wird im Weiteren ein viertes Ausführungsbeispiel eines Sensor-Feldes einer erfindungsgemäßen Sensor-Anordnung beschrieben.

Das in Fig.5B gezeigte Sensor-Feld 510 ist in einem Kreuzungsbereich einer Zeilen-Leitung 511 und einer ersten Spalten-Leitung 512a angeordnet. Wie im Falle des Sensor-Feldes 500 weist auch das Sensor-Feld 510 einen Detektions-Transistor 513 auf. Darüber hinaus weist die Kopplungs-Einrichtung des Sensor-Felds 510 eine Kalibrier-Einrichtung zum Kalibrieren der Kopplungs-Einrichtung auf. Gemäß dem in Fig.5B gezeigten Ausführungsbeispiel weist die Kalibrier-Einrichtung einen Kalibrier-Transistor 515 mit einem mit der Zeilen-Leitung 511 gekoppelten ersten Source-/Drain-Anschluss, mit einem mit dem Gate-Anschluss des Detektions-Transistors 513 sowie mit einem mit dem zugeordneten Sensor-Element 514 gekoppelten Kondensator 516 gekoppelten zweiten Source-/Drain-Anschluss und mit einem mit einer zweiten Spalten-Leitung 512b gekoppelten Gate-Anschluss auf, wobei mittels der zweiten Spalten-Leitung 512b an den Gate-Anschluss des Kalibrier-Transistors 515 eine elektrische Kalibrier-Spannung anlegbar ist.

Die Kalibrier-Einrichtung des Sensor-Felds 510 ist derart eingerichtet, dass mittels geeigneten Steuerns der Spannungssignale an der ersten und der zweiten Spalten-Leitung 512a, 512b sowie an der Zeilen-Leitung 511 eine Abweichung von Parametern des Detektions-Transistors 513 von Parametern von Detektions-Transistoren anderer Sensor-Felder der erfindungsgemäßen Sensor-Anordnung aufgrund von Ungleichmäßigkeiten bei dem Herstellungsverfahren kompensiert werden kann. Insbesondere kann eine statistische Streuung des Wertes der Schwellenspannung der Detektions-Transistoren 513 unterschiedlicher Sensor-Felder einer Sensor-Anordnung um einen mittleren Wert auftreten. Die Abweichung der Schwellenspannung zwischen unterschiedlichen Sensor-Feldern kann dadurch kompensiert werden, dass die zweite Spalten-Leitung 512b auf ein solches elektrisches Potential gebracht wird, dass der Kalibrier-Transistor 515 leitend ist und der elektrische Knoten zwischen dem Kondensator 516 und dem Gate-Anschluss des Detektions-Transistors 513 auf ein Kalibrierungs-Potential gebracht wird. Das Kalibrierungs-Potential ist durch den in die Zeilen-Leitung 511 eingespeisten elektrischen Strom determiniert, welcher durch den als Diode geschalteten Detektions-Transistor 513 fließt. Ist der Kalibrier-Transistor 515 wieder nichtleitend, verbleibt auf dem Gate-Anschluss des Detektions-Transistors 513 eine elektrische Spannung, mittels der eine Korrektur der unterschiedlichen Schwellenspannungen unterschiedlicher Detektions-Transistoren 513 unterschiedlicher Sensor-Felder einer Sensor-Anordnung ermöglicht ist.

Im Weiteren wird bezugnehmend auf **Fig.5C** ein fünftes Ausführungsbeispiel eines Sensor-Felds der erfindungsgemäßen Sensor-Anordnung beschrieben.

Das Sensor-Feld 520 weist wie das Sensor-Feld 510 die folgenden, analog zu der in Fig.5B gezeigten Weise, verschalteten Komponenten auf: eine Zeilen-Leitung 521, eine erste und eine zweite Spalten-Leitung 522a, 522b, einen Detektions-Transistor 523, ein Sensor-Element 524, einen Kalibrier-Transistor 525 und einen Kondensator 526. Darüber hinaus weist das Sensor-Feld 520 ein Verstärker-Element zum Verstärken des elektrischen Einzel-Stromflusses der Kopplungs-Einrichtung des Sensor-Feldes 520 auf. Dieses Verstärker-Element ist als Bipolar-Transistor 527 mit einem mit der Zeilen-Leitung 521 gekoppelten Kollektor-Anschluss, mit einem mit der ersten Spalten-Leitung 522a gekoppelten Emitter-Anschluss und mit einem mit dem zweiten Source-/Drain-Bereich des Detektions-Transistors 523 gekoppelten Basis-Anschluss auf. Der elektrische Strom zwischen der Zeilen-Leitung 521 und der ersten Spalten-Leitung 522a wird aufgrund der stromverstärkenden Wirkung des Bipolar-Transistors 527 stark verstärkt. Dadurch wird eine erhöhte Empfindlichkeit der gesamten Sensor-Anordnung erreicht.

In **Fig.5D** ist ein Sensor-Feld 530 gemäß einem sechsten Ausführungsbeispiel der Erfindung gezeigt.

Das Sensor-Feld 530 ist wabenförmig ausgebildet. Eine Zeilen-Leitung 531 schließt mit einer ersten Spalten-Leitung 532a und mit einer zweiten Spalten-Leitung 532b jeweils einen Winkel von 60° ein, wobei auch die beiden Spalten-Leitungen 532a und 532b einen Winkel von 60° miteinander einschließen. Das Sensor-Feld 530 weist einen ersten Detektions-Transistor 533a und einen zweiten Detektions-Transistor 533b auf. Die Gate-Anschlüsse der beiden Detektions-Transistoren 533a, 533b sind mit einem Sensor-Element 534 gekoppelt. Der erste Source-/Drain-Anschluss des ersten Detektions-Transistors 533a und der erste Source-/Drain-Anschluss des zweiten Detektions-Transistors 533b sind mit der Zeilen-Leitung 531 gekoppelt. Der zweite Source-/Drain-Anschluss des ersten Detektions-Transistors 533a ist mit der ersten Spalten-Leitung 532a gekoppelt, wohingegen der zweite Source-/Drain-Anschluss des zweiten Detektions-Transistors 533b mit der zweiten Spalten-Leitung 532b gekoppelt ist.

Findet an dem Sensor-Element 534 ein Sensor-Ereignis statt, wodurch an dem Sensor-Element 534 elektrische Ladungsträger erzeugt werden, so ändert sich dadurch die Leitfähigkeit der Kanal-Bereiche des ersten und des zweiten Detektions-Transistors 533a, 533b in charakteristischer Weise. Dadurch verändert sich einerseits der elektrische Stromfluss von der Zeilen-Leitung 531 in die erste Spalten-Leitung 532a und andererseits der Stromfluss von der Zeilen-Leitung 531 in die zweite Spalten-Leitung 532b. Auch gemäß dem in Fig.5D gezeigten Konzept werden die Summen-Stromflüsse in den Spalten-Leitungen und in den Zeilen-Leitungen in Randbereichen einer Anordnung einer Vielzahl von Sensor-Feldern 530 erfasst und über die zeitliche Korrelation der Summen-Stromflüsse die Signale der einzelnen Sensor-Felder 530 berechnet.

Da aufgrund der platzsparenden Ausgestaltung der bezugnehmend auf Fig.4A bis Fig.5D gezeigten Sensor-Felder die Sensor-Felder ausreichend klein ausgebildet werden können, um eine hohe Orts-Auflösung zu erreichen, kann der Rauschpegel in dem Einzelstrom eines Sensor-Feldes einen Wert annehmen, der in der gleichen Größenordnung wie der eigentliche Signalstrom liegen kann. Auf den Zeilen-Leitungen beziehungsweise den Spalten-Leitungen addieren sich zwar die Rausch-Stromflüsse aller angeschlossenen Sensor-Elemente auf, dieses unkorrelierte Signal fällt jedoch bei Korrelationsrechnung heraus, so dass nur das Sensor-Signal und das Rausch-Signal eines einzigen Sensor-Feldes zum berechneten Messsignal dieses Sensor-Feldes beiträgt.

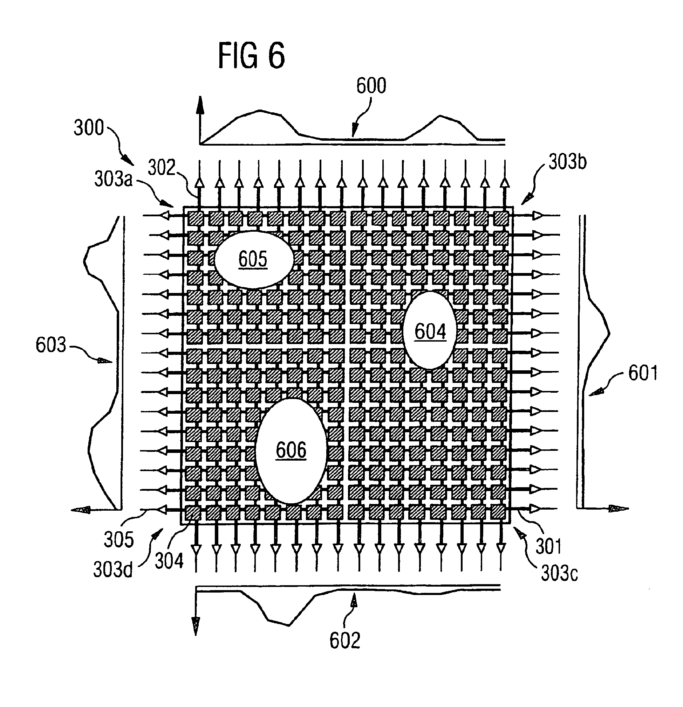

Im Weiteren wird bezugnehmend auf **Fig.6** die in Fig.3 gezeigte Sensor-Anordnung 300 in einem aktiven Betriebszustand beschrieben.

Gemäß dem in Fig.6 gezeigten Betriebszustand der Sensor-Anordnung 300 sind ein erstes Neuron 604, ein zweites Neuron 605 und ein drittes Neuron 606 auf der matrixförmigen Anordnung von Sensor-Feldern 304 angeordnet. Die Sensor-Felder 304 sind gemäß dem bevorzugten Ausführungsbeispiel elektrisch leitfähige Elektroden (z.B. Au, Pt, Pd), die mit einem Dielektrikum beschichtet (z.B. SiO₂, Si₃N₄) sind und in elektrischer Wirkverbindung mit einem Verstärker (z.B. MOSFET) sind. Ferner in Fig.6 gezeigt ist eine erste Projektion 600, eine zweite Projektion 601, eine dritte Projektion 602 und eine vierte Projektion 603 der zweidimensionalen Anordnung von Neuronen 604 bis 606 auf der matrixförmigen Sensor-Anordnung 300. Wie bezugnehmend auf Fig.3 beschrieben, ist die matrixförmige Sensor-Anordnung 300 in vier Teil-Bereiche 303a bis 303d aufgeteilt, die jeweils mit eigenen Zeilen- beziehungsweise Spalten-Leitungen gekoppelt sind. Daher liefern die Projektionen 600 bis 603 jeweils ein zweidimensionales Abbild der Anordnung von ein Sensor-Signal generierenden Neuronen in den jeweiligen TeilBereichen 303a bis 303d. Beispielsweise liefert das erste Neuron 604, das im Wesentlichen in dem zweiten Teil-Bereich 303b der Sensor-Anordnung 300 angeordnet ist, ein entsprechendes Signal in dem gemäß Fig.6 rechten Teil-Bereich der ersten Projektion 600 und in dem mittleren Bereich der zweiten Projektion 601. Da das erste Neuron 604 zu einem geringen Teil auch in dem dritten Teil-Bereich 303c angeordnet ist, ist in dem gemäß Fig.6 rechten Teil-Bereich der dritten Projektion 602 ein geringes Signal des ersten Neurons 604 zu sehen. Auf diese Weise trägt jedes der Neuronen 604 bis 606 in jeweils einem Teil der Projektionen 600 bis 603 zu einem Signal bei. Die kombinierten Signale der Projektionen 600 bis 603 liefern Informationen über die räumliche Anordnung der Neuronen 604 bis 606.

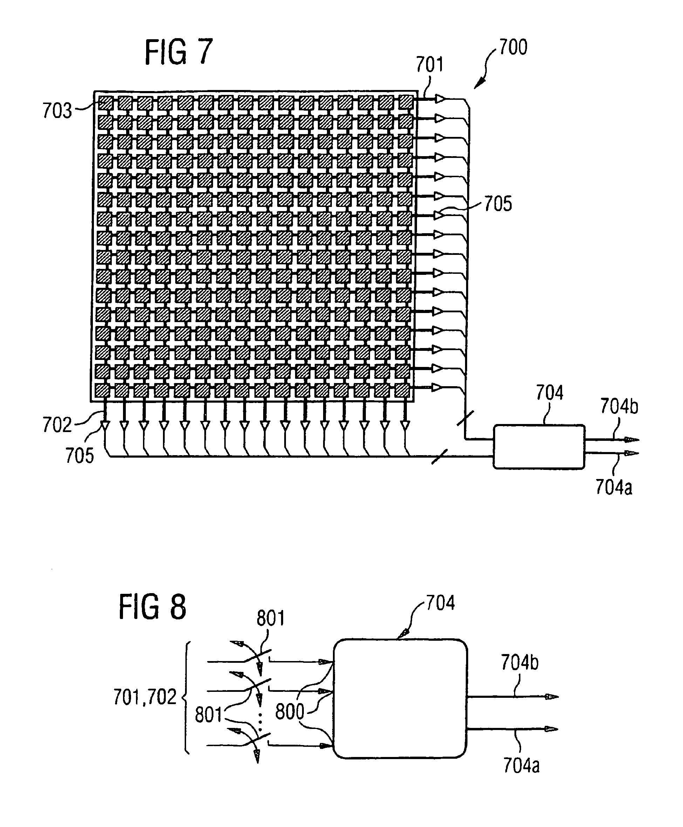

Im Weiteren wird bezugnehmend auf **Fig.7** ein drittes bevorzugtes Ausführungsbeispiel der erfindungsgemäßen Sensor-Anordnung beschrieben.

Die in Fig.7 gezeigte Sensor-Anordnung 700 weist sechzehn horizontal angeordnete Zeilen-Leitungen 701, sechzehn vertikal angeordnete Spalten-Leitungen 702 und 256 in den Kreuzungsbereichen der Zeilen-Leitungen 701 mit den Spalten-Leitungen 702 angeordnete Sensor-Felder 703 auf. Jedes der Sensor-Felder 703 ist so ausgebildet wie das in Fig.5A gezeigte Sensor-Feld 500. An den jeweiligen End-Abschnitten der Zeilen-Leitungen 701 und der Spalten-Leitungen 702 sind elektrisch gekoppelte Mittel zum Erfassen eines jeweiligen Summen-Stromflusses aus den von den Sensor-Feldern 703 der jeweiligen Leitung 701, 702 bereitgestellten elektrischen Einzel-Stromflüssen vorgesehen. Gemäß dem in Fig.7 gezeigten Ausführungsbeispiel der Sensor-Anordnung 700 sind diese Mittel Teil einer als Maximum-Likelihood Sequence Estimation Dekoder ausgestalteten Dekodier-Einrichtung 704. Die mit den Zeilen-Leitungen 701 und den Spalten-Leitungen 702 gekoppelte Dekodier-Einrichtung 704 ist derart eingerichtet, dass sie aus zumindest einen Teil der elektrischen Summen-Stromflüsse, welche der Dekodier-Einrichtung 704 über die Zeilen-Leitungen 701 und die Spalten-Leitungen 702 zuführbar sind, diejenigen Sensor-Elemente der Sensor-Felder 703 ermittelt, an denen ein Sensor-Signal anliegt.

Ferner weist jede Zeilen-Leitung 701 und jede Spalten-Leitung 702 eine Verstärker-Einrichtung 705 zum Verstärken und optional eine Abtast-/Halte-Einrichtung (nicht gezeigt) zum zeitgenauen Abspeichern des in der jeweiligen Zeilen-Leitung 701 bzw. Spalten-Leitung 702 fließenden elektrischen Summen-Stromflusses auf.

Bevor die in Fig.7 gezeigte Sensor-Anordnung 700 näher beschrieben wird, wird nochmals auf die in Fig.6 gezeigte Sensor-Anordnung 300 Bezug genommen. Der bezugnehmend auf Fig.6 beschriebene Betrieb der Sensor-Anordnung 300 zur Ermittlung des räumlichen und zeitlichen Verlaufs von Sensor-Ereignissen mittels Berechnens paarweiser Kreuzkorrelationen liefert sehr gute Ergebnisse, wenn, wie in Fig.6 gezeigt, nur ein geringer Anteil der Sensor-Felder ein Sensor-Signal liefert.

Bezugnehmend auf Fig.6 wird eine Weiterbildung der erfindungsgemäßen Sensor-Anordnung beschrieben, bei der auch bei einer starken Überlappung von Sensor-Signalen der einzelnen Sensor-Felder oder bei Vorliegen starken Rauschens eine Dekodierung und daher eine Bestimmung der Verläufe der einzelnen Sensor-Signale ermöglicht ist. Damit ist sichergestellt, dass auch bei Vorliegen der beschriebenen Störeinflüsse aus den Summen-Stromflüssen der Zeilen- bzw. Spalten-Leitungen die einzelnen Sensor-Signale rekonstruiert werden können. Dies erfolgt auf der Basis von zu Beginn der Dekodierung vorbekannter Informationen über mögliche Signalverläufe. Für viele naturwissenschaftlich-technische Prozesse sind charakteristische Signalverläufe bekannt.

Bei der Sensor-Anordnung 300 sind matrixförmig angeordnete Sensor-Felder 304 an gemeinsame Zeilen- und Spalten-Leitungen 301, 302 angeschlossen. Auf diesen gemeinsamen Zeilen- bzw. Spalten-Leitungen 301, 302 addieren sich die Einzel-Stromflüsse aller angeschlossenen Sensor-Felder. Mittels Berechnens paarweiser Kreuzkorrelationen der zeitabhängigen Summen-Stromflüsse aller Spalten mit allen Zeilen unter Verwendung einer Fourier-Transformation und einer Fourier-Rücktransformation ist die Bestimmung der Sensor-Signale jedes einzelnen Sensor-Feldes ermöglicht. Dieses Konzept ermöglicht bei gleichzeitiger Aktivität nur eines Sensor-Feldes dessen genaue Ortung selbst in Anwesenheit von RauschSignalen der übrigen Sensor-Felder. Das beschriebene Konzept ist besonders vorteilhaft bei Sensor-Anordnungen mit einer hohen Anzahl von Zeilen und Spalten, bei der nur ein geringer Anteil der Sensor-Felder zu einem festen Zeitpunkt ein Sensor-Ereignis aufweist. Dann ist es ohne eine zusätzliche Auswahllogik zur ortsaufgelösten Messung der Signale ausreichend, die Summen-Stromflüsse entlang der Zeilen-Leitungen und Spalten-Leitungen zu erfassen und die Sensor-Signale der Sensor-Felder rechnerisch zu ermitteln.

Allerdings kann es in der Praxis vorkommen, dass mehrere Sensor-Felder gleichzeitig ein Sensor-Signal liefern und dass die von ihnen generierten Sensor-Signale ähnliche oder gleiche Intensitäten aufweisen. Gemäß dem Konzept der Sensor-Anordnung 300 werden die von den Sensor-Feldern generierten Signale sowohl zeilen- als auch spaltenweise überlagert. Probleme können sich ergeben, wenn gleichzeitig mehrere Sensor-Felder aktiv sind, deren Einzel-Stromflüsse entlang der Zeilen- und Spalten-Leitungen aufsummiert werden. Dann kann es vorkommen, dass der Wert der Kreuzkorrelierten nicht in jedem Fall für genau ein Spalten- und Zeilensignal den maximal möglichen Wert annimmt, sondern unter Umständen für andere, überlagerte Zeilen- und Spaltensignale mit hoher Intensität. In diesen Fällen ist eine eindeutige Detektion der einzelnen Sensor-Signale zumindest erschwert.

Diese Schwierigkeiten sind gemäß dem in Fig.7 gezeigten Konzept umgangen, indem die Dekodier-Einrichtung 704 als Maximum-Likelihood Sequence Estimation-Dekoder ausgestaltet ist. Die Funktionalität der Dekodier-Einrichtung 704 wird im Weiteren beschrieben.

Bei der Rückberechnung der Sensor-Signale der Sensor-Felder 703 aus den Summen-Stromflüssen der Zeilen-Leitungen 701 bzw. der Spalten-Leitungen 702 werden gemäß der Funktionalität der Dekodier-Einrichtung 704 bezüglich eines bestimmten Sensor-Feldes 703 in der i-ten Zeile und der j-ten Spalte der matrixförmigen Anordnung von Sensor-Feldern 703 nicht nur die Information der Summen-Stromflüsse der i-ten Zeile und der j-ten Spalte verwendet, sondern es werden zusätzlich Informationen aus anderen, vorzugsweise allen anderen, Spalten 1≠j und Zeilen k≠i verwendet. Bei einer Überlagerung von einzelnen, sich im Allgemeinen zeitlich überlagernden Sensor-Signalen besteht nämlich nicht nur eine Abhängigkeit zwischen genau einem Summen-Stromfluss einer Zeilen-Leitung und einem Summen-Stromfluss einer Spalten-Leitung, sondern diese Abhängigkeiten erstrecken sich über größere Bereiche aktiver Zeilen-Leitungen und Spalten-Leitungen. Diese Wechselwirkungen werden in das von der Dekodier-Einrichtung 704 durchgeführte Auswerteverfahren miteinbezogen, indem auch Summen-Stromflüsse von Zeilen- bzw. Spalten-Leitungen, in deren Kreuzungsbereichen ein betrachtetes Sensor-Feld 703 nicht angeordnet ist, in das Ermittlungsverfahren miteinbezogen. Mit anderen Worten ist die Dekodier-Einrichtung 704 derart eingerichtet, dass zum Ermitteln, ob an einem Sensor-Element ein Sensor-Signal anliegt, mindestens ein Summen-Stromfluss mindestens einer nebenliegenden Zeilen-Leitung und/oder mindestens einer nebenliegenden Spalten-Leitung verwendet wird.

Ferner weist die Dekodier-Einrichtung 704 die Funktionalität auf, dass bei dem Ermitteln der Sensor-Felder, an denen ein Sensor-Signal anliegt, eine erwartete Kurvenform der Signale, die aufgrund physikalischer Randbedingungen in den einzelnen Sensor-Feldern generiert werden können, berücksichtigt werden. So senden beispielsweise Nervenzellen als Reaktion auf eine elektrische Anregung ein Signal mit einer charakteristischen Kurvenform aus. Werden solche Randbedingungen bei dem Ermittlungsverfahren berücksichtigt, so kann das Ermitteln von denjenigen Sensor-Feldern 703, an denen ein Sensor-Signal anliegt, ausreichend sicher durchgeführt werden. Daher ist die Dekodier-Einrichtung 704 derart eingerichtet, dass zum Ermitteln, ob an einem Sensor-Element ein Sensor-Signal anliegt, mindestens ein vorgegebenes zeitliches und/oder räumliches Referenzsignal verwendet wird.

Indem vorbekannte Informationen (beispielsweise bezüglich zu erwartender Kurvenformen von Signalen) verwendet werden, kann auch die Überlagerung unterschiedlicher Signale mit der Signalform eines Referenzsignals berücksichtigt werden. Hierzu kann ein Algorithmus verwendet werden, der derart eingerichtet ist, das er aus einem erfassten Signal, dass aus einer Mehrzahl von Einzelsignalen zusammengesetzt ist, dieses Signal in die Teilkomponenten zerlegt. Insbesondere der Maximum-Likelihood Sequence Estimation-Algorithmus und der Maximum a posteriori-Algorithmus sind hierfür geeignet. Wie oben beschrieben, ist die Dekodier-Einrichtung 704 als Maximum-Likelihood Sequence Estimation-Dekoder ausgestaltet.

Es ist zu betonen, dass die Mustersignale bzw. Referenzsignale nicht im Detail vorbekannt sein müssen. Die Muster- bzw. Referenzsignale können an die tatsächlich ermittelten Signale adaptiert, d.h. angepasst werden. Zusammenfassend weist daher die Dekodier-Einrichtung 704 die Funktionalität auf, dass zum Ermitteln, ob an einem Sensor-Element ein Sensor-Signal anliegt, das mindestens eine vorgegebene zeitliche und/oder räumliche Referenzsignal an das erfasste Signal angepasst wird. Darüber hinaus ist die Dekodier-Einrichtung 704 derart eingerichtet, dass zum Ermitteln, ob an einem Sensor-Element zu einem zweiten Zeitpunkt ein Sensor-Signal anliegt, eine vorgegebene Referenzinformation über Sensor-Signale zu einem ersten Zeitpunkt verwendet wird, der zeitlich vor dem zweiten Zeitpunkt liegt.

Mit anderen Worten wird von der Dekodier-Einrichtung 704 eine Entscheidung über die Aktivität eines Sensor-Feldes unter Berücksichtigung von mindestens einem Referenzsignal getroffen, das einen zu erwartenden Signalverlauf charakterisiert. Parameter des Referenzsignals werden an den tatsächlichen Signalverlauf angepasst und an einem Ausgang 704a der Dekodier-Einrichtung 704 für eine Weiterverarbeitung bereitgestellt. Ferner ist die Dekodier-Einrichtung 704 derart eingerichtet, dass eine zeitliche und/oder räumliche Überlagerung mehrerer Sensor-Signale in den Entscheidungsprozess einbezogen werden kann. Im Falle der Dekodier-Einrichtung 704 wird bei dem Entscheidungsprozess der Maximum Likelihood Sequence Estimation-Algorithmus verwendet.

Wie in Fig.7 gezeigt, weist die Dekodier-Einrichtung 704 den ersten Ausgang 704a und ferner einen zweiten Ausgang 704b auf. Der erste Ausgang 704a ist derart eingerichtet, dass an diesem Referenzsignale (Mustersignale) bereitgestellt werden können. An dem zweiten Ausgang 704b können Datensignale bereitgestellt werden, in denen die Information enthalten ist, an welchen der Sensor-Felder 703 mit hoher Wahrscheinlichkeit ein Sensor-Ereignis stattgefunden hat.

In **Fig.8** ist der schematische Aufbau der Dekodier-Einrichtung 704 gezeigt. Die Dekodier-Einrichtung 704 weist eine Mehrzahl von Eingangs-Schnittstellen 800 auf. Jede der Zeilen-Leitungen 701 und jede der Spalten-Leitungen 702 ist jeweils mit genau einer der Eingangs-Schnittstellen 800 der Dekodier-Einrichtung 704 gekoppelt. Zwischen einem End-Abschnitt jeder Zeilen-Leitung 701 bzw. jeder Spalten-Leitung 702 einerseits und der jeweils zugeordneten Eingangs-Schnittstelle 800 der Dekodier-Einrichtung 704 andererseits ist jeweils ein Schalter 801 angeordnet, der in einem offenen und in einem geschlossenen Zustand vorliegen kann. Um den Summen-Stromfluss einer Zeilen-Leitung 701 bzw. einer Spalten-Leitung 702 über die jeweils zugeordnete Eingangs-Schnittstelle der Dekodier-Einrichtung 704 zuzuführen, wird der der jeweiligen Leitung zugeordnete Schalter 801 geschlossen. Die der Dekodier-Einrichtung 704 zugeführten Summen-Stromflüsse der Zeilen-Leitungen 701 bzw. der Spalten-Leitungen 702 werden in der Dekodier-Einrichtung 704 ausgewertet, wobei unter Verwendung des Maximum-Likelihood Sequence Estimation-Algorithmus eine Schätzung der wahrscheinlichsten Aktivität der Sensor-Felder 703 durchgeführt wird. Die zeitliche Abfolge, mit der die einzelnen Schalter 801 sukzessive geöffnet und wieder geschlossen werden, hängt von der Bandbreite des eingehenden Signals und von der Wahrscheinlichkeit ab, dass mehr als ein Sensor-Feld 703 im gleichen Abtastintervall denselben Signalverlauf generiert. Diese Wahrscheinlichkeit nimmt unter der Annahme einer Gleichverteilung proportional mit der Länge des Abtastintervalls zu. Eine erhöhte Abtastrate ist daher wünschenswert und bietet eine Möglichkeit zur Systemoptimierung.

Nochmals bezugnehmend auf Fig.8 kann die Funktionalität der Dekodier-Einrichtung 704 wie folgt zusammengefasst werden. Bei einem geschlossenen Schalter 801 wird der Dekodier-Einrichtung 704 ein Summen-Stromfluss der dem geschlossenen Schalter zugeordneten Zeilen-Leitung 701 bzw. Spalten-Leitung 702 bereitgestellt. Dadurch werden der Dekodier-Einrichtung 704 sukzessive die Summen-Stromflüsse aller Zeilen-Leitungen 701 und aller Spalten-Leitungen 702 bereitgestellt. Diese Summen-Stromflüsse sind ein Rausch-Signal aufweisende, kodierte Signale, die ein Abbild von den Sensor-Ereignissen auf den Sensor-Feldern 703 der Sensor-Anordnung 700 sind. Auf Grund der Funktionalität der als Maximum-Likelihood Sequence Estimation-Dekoder ausgestalteten Dekodier-Einrichtung 704 wird rechnerisch aus den Summen-Stromflüssen eine Entscheidung über das Vorliegen bzw. Nichtvorliegen eines Sensor-Ereignisses an den Sensor-Feldern 703 getroffen, die von allen berechneten Konstellationen gemäß dem Maximum-Likelihood Sequence Estimation-Verfahren am wahrscheinlichsten den tatsächlichen Sensor-Ereignissen entsprechen.

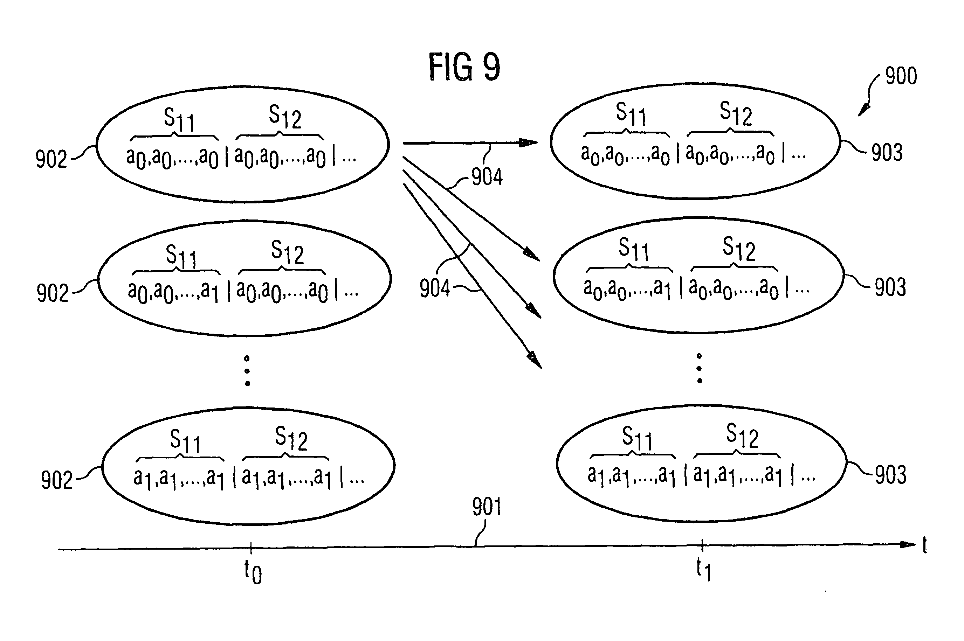

Im Weiteren wird das in **Fig.9** gezeigte Trellis-Diagramm 900 beschrieben.

Das Trellis-Diagramm 900 ist ein entlang einer Zeitachse 901 aufgetragener Zustandsübergangs-Graph eines zu beschreibenden Systems. Entlang der Zeitachse 901 sind Zustände der Sensor-Felder der erfindungsgemäßen Sensor-Anordnung zu unterschiedlichen Zeitpunkten dargestellt. Zu einem festen Zeitpunkt, beispielsweise zu dem in Fig.9 gezeigten Zeitpunkt t₀ sind orthogonal zu der Zeitachse 901 mögliche Zustände der Sensor-Anordnung, genauer gesagt der n m Sensor-Felder der Sensor-Anordnung, dargestellt. Dabei bezeichnet m die Zahl der Zeilen der matrixförmigen Anordnung von Sensor-Feldern und n die Anzahl der Spalten. Mit sᵢⱼ ist das Sensor-Feld der i-ten Zeile und der j-ten Spalte der matrixförmigen Sensor-Anordnung bezeichnet. Die Zustände der n·m-dimensionalen Sensor-Anordnung lassen sich in einem Trellis-Diagramm mit A^{(nmL)} Zuständen beschreiben, wobei (A-1) die Anzahl und (L+1) die Länge der Musterimpulse (Referenzsignale) aₖ bezogen auf das Abtastintervall bezeichnet. Der zu einem bestimmten Zeitpunkt, beispielsweise t₀, aktuelle Ausgangswert jedes der n m Sensor-Felder sᵢⱼ hängt von L früheren Aktivitätszuständen ab, wobei es A unterschiedliche Möglichkeiten a₀, a₁,..., a_{A} gibt (keine Aktivität oder ein Mustersignal aus (A-1) unterschiedlichen).

Pfeile 904 zeigen mögliche Übergänge zwischen Trellis-Zuständen zu einem ersten Zeitpunkt t₀ 902 und Trellis-Zuständen zu einem zweiten Zeitpunkt t₁ 903 an. Da von einem Abtastintervall zum nächsten die Anzahl der möglichen Zustandsänderungen, d.h. die Anzahl der Änderungen im Aktivitätsmuster, eines jeden Sensors auf höchstens A Möglichkeiten beschränkt sind, ergeben sich insgesamt (A·n·m) mögliche Zustandänderungen im Trellis-Diagramm 900. Dies entspricht der Anzahl der von jedem Zustand ausgehenden möglichen Übergänge, welche durch die Pfeile 904 angedeutet sind. Die Anzahl der möglichen Übergänge verringert sich, wenn vorbekannte Informationen hinsichtlich von Referenzsignalen über die zeitlichen/räumlichen Verläufe der Sensor-Signale vorbekannt sind und verwendet werden können.

Eine basierend auf dem Trellis-Diagramm 900 operierende, als Maximum-Likelihood Sequence Estimation-Dekoder ausgestaltete Dekodier-Einrichtung liefert zu jedem Abtastintervall eine Schätzung über den Zustand des Sensor-Feldes, d.h. den Aktivitätsmuster der einzelnen Sensor-Felder. Aus diesen Daten und aus dem ursprünglichen Aktivitätsmuster können die tatsächlichen Mustersequenzen berechnet werden. Diese können für eine andere Messung oder für eine Neuauswertung der aktuellen Messung verwendet werden (iterativ).

Anschaulich wird unter Verwendung des Maximum-Likelihood Sequence Estimation-Verfahrens derjenige Pfad von Trellis-Zuständen 902, 903, ... entlang der Zeitachse 901 ermittelt, der dem wahrscheinlichsten Signalverlauf entspricht.

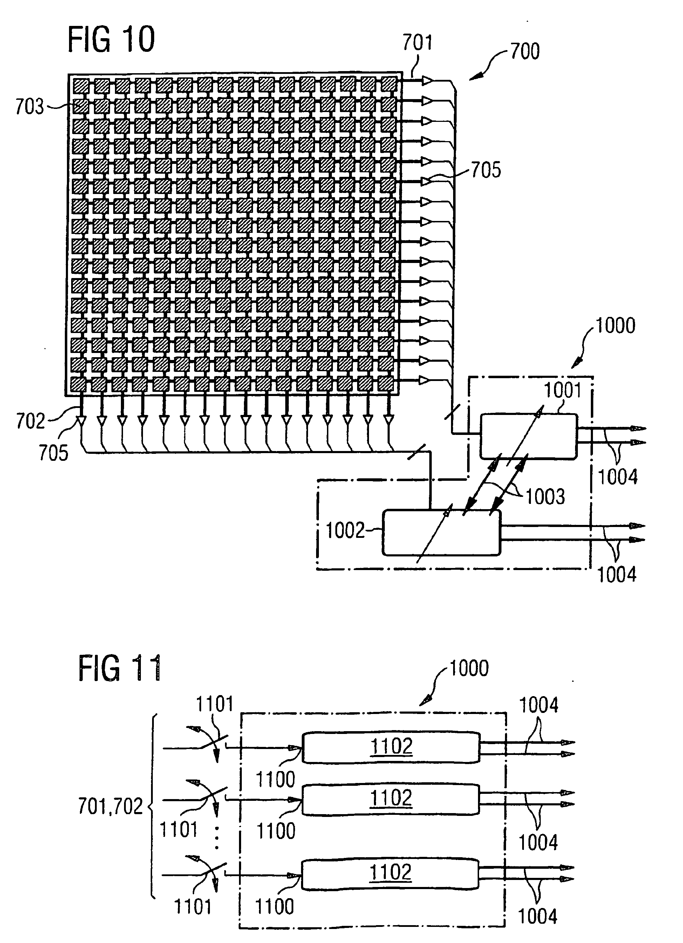

Im Weiteren wird bezugnehmend auf **Fig.10** eine Sensor-Anordnung gemäß einem vierten Ausführungsbeispiel der Erfindung beschrieben.

Die in Fig.10 gezeigte Sensor-Anordnung 700 weist abweichend von der in Fig.7 gezeigten Ausgestaltung eine Dekodier-Einrichtung 1000 auf. Die Dekodier-Einrichtung 1000 ist als Maximum-Likelihood Sequence Estimation-Dekoder ausgestaltet.

Die Dekodier-Einrichtung 1000 ist in eine Zeilendekodier-Einrichtung 1001, der die elektrischen Summen-Stromflüsse der Zeilen-Leitungen 701 zuführbar sind, und in eine Spaltendekodier-Einrichtung 1002, der die elektrischen Summen-Stromflüsse der Spalten-Leitungen 702 zuführbar sind, aufgeteilt. Die Zeilendekodier-Einrichtung 1001 ist derart eingerichtet, dass aus den elektrischen Summen-Stromflüssen der Zeilen-Leitungen 701 unabhängig von den Summen-Stromflüssen der Spalten-Leitungen 702 Informationen über diejenigen Sensor-Elemente 703 ermittelbar sind, an denen möglicherweise ein Sensor-Signal anliegt. Die Spaltendekodier-Einrichtung 1002 ist derart eingerichtet, dass aus den elektrischen Summen-Stromflüssen der Spalten-Leitungen 702 Informationen über diejenigen Sensor-Elemente 703 ermittelbar sind, an denen möglicherweise ein Sensor-Signal anliegt. Die Dekodier-Einrichtung 1000 ist ferner derart eingerichtet, dass mittels gemeinsamen Auswertens der von der Zeilendekodier-Einrichtung 1001 und der Spaltendekodier-Einrichtung 1002 ermittelten Informationen diejenigen Sensor-Elemente ermittelbar sind, an denen ein Sensor-Signal anliegt.

Mit anderen Worten werden die Summen-Stromflüsse der Zeilen-Leitungen 701 ausgewertet, ohne dass in diesem Auswerteprozess die Informationen über die Summen-Stromflüsse der Spalten-Leitungen 702 berücksichtigt sind. Analog werden in der Spaltendekodier-Einrichtung 1002 die Summen-Stromflüsse der Spalten-Leitungen 702 unabhängig von den Summen-Stromflüssen der Zeilen-Leitungen 701 ausgewertet. Dies führt zu einer erheblichen numerischen Vereinfachung.

Die beschriebene Dekodier-Einrichtung 1000 weist den Vorteil auf, dass die bei Sensor-Anordnungen mit einer hohen Anzahl von Sensor-Feldern numerisch sehr aufwändige, gemeinsame Auswertung der Summen-Stromflüsse der Zeilen- und der Spalten-Leitungen vermieden ist. Die Funktionalität der Dekodier-Einrichtung 1000 basiert auf einem suboptimalen, iterativen Verfahren zum Ermitteln von denjenigen Sensor-Feldern 703, an denen ein Sensor-Signal anliegt. Im Gegensatz zu der Dekodier-Einrichtung 704 aus Fig.8 werden die Summen-Stromflüsse der Zeilen-Leitungen 701 in der Zeilendekodier-Einrichtung 1001 einerseits, und die Summen-Stromflüsse der Spalten-Leitungen 702 mittels der Spaltendekodier-Einrichtung 1002 zunächst unabhängig voneinander dekodiert. Obwohl der prinzipielle Ablauf des diesem Dekodieren zugrunde liegenden Algorithmus dem oben bezugnehmend auf Fig.7, Fig.8, Fig.9 beschriebenen Verfahren entspricht, besitzt das Trellis-Diagramm im Falle der Dekodier-Einrichtung 1000 jeweils nur A^{L} Zustände und entsprechend weniger Übergänge zwischen Trellis-Zuständen zu unterschiedlichen Zeitpunkten. Somit ist die Dekodierung schneller und mit einem geringeren Rechenaufwand möglich. Mittels Auffindens gleichzeitiger Aktivierungsmuster der Sensoren kann durch Abgleich der Zeilen und Spaltensignale die Position der aktiven Sensor-Felder 703 bestimmt werden. Ein solcher Abgleich ist in Fig.10 mittels der Pfeile 1003 symbolisiert. Die in Fig.10 gezeigten Ausgangssignale 1004 können Daten über aktivierte Sensor-Felder 703 und Referenzsignale (Mustersignale) aufweisen.

Auch im Falle der Dekodier-Einrichtung 1000 ist es möglich, dass verwendete Mustersequenzen tatsächlich gemessenen Sequenzen angepasst werden. Nach der Übertragung der Mustersignale kann der Detektions-Prozess mit denselben Sensordaten erneut angestoßen werden. Dann werden aber bereits geeignetere, da in dem vorhergehenden Schritt angepasste Mustersignale verwendet, so dass durch dieses Verfahren die Fehlertoleranz gegenüber Rauschen mit jedem Iterationsschritt verbessert wird. Die Anzahl der Iterationsschritte ist erfindungsgemäß vorgebbar, so dass mittels Auswählens der Anzahl der Iterationsschritte die Genauigkeit des Dekodierverfahrens einstellbar ist.

Im Weiteren wird bezugnehmend auf **Fig.11** der schematische Aufbau einer Ausgestaltung der Dekodier-Einrichtung 1000 beschrieben.

Wie in Fig.11 gezeigt, weist die Dekodier-Einrichtung 1000 n+m Eingangs-Schnittstellen 1100 auf. Zwischen jeweils einer der Eingangs-Schnittstellen 1100 und einem End-Abschnitt der einer jeden Eingangs-Schnittstelle 1100 zugeordneten genau einen Zeilen- bzw. Spalten-Leitung 701, 702 ist ein Schalter 1101 angeordnet. Die Dekodier-Einrichtung 1000 ist in n+m Teil-Dekodier-Einrichtungen 1102 aufgeteilt, von denen jede in einem Szenario, in dem der jeweils zugehörige Schalter 1001 geschlossen ist, mit einer der Zeilen-Leitungen 701 bzw. der Spalten-Leitungen 702 gekoppelt ist, so dass der jeweiligen Eingangs-Schnittstelle 1100 der zugehörige Summen-Stromfluss zuführbar ist. Mit anderen Worten wird jeweils ein Summen-Stromfluss einer der m Zeilen-Leitungen 701 bzw. der n Spalten-Leitungen 702 einer der Teil-Dekodier-Einrichtungen 1102 bereitgestellt. Daher bilden m der Teil-Dekodier-Einrichtungen 1102 die Zeilendekodier-Einrichtung 1101, wohingegen m der Teil-Dekodier-Einrichtung 1102 die Spaltendekodier-Einrichtung 1002 bilden. Die Summen-Stromflüsse der Zeilen-Leitungen 701 bzw. die Summen-Stromflüsse der Spalten-Leitungen 702 werden in den jeweiligen Teil-Dekodier-Einrichtungen 1102 voneinander unabhängig dekodiert. An den Ausgängen der Dekodier-Einrichtung 1000 sind die Ausgangssignale 1004 bereitgestellt, die Daten über aktivierte Sensor-Felder 703 aufweisen. Ferner können die Ausgangssignale 1004 Informationen über die Zuverlässigkeit der Auswertung bzw. Mustersequenzen enthalten.

In diesem Dokument sind folgende Veröffentlichungen zitiert:
[1] WO 00/62048 A2
[2] US 05965452 A1
[3] US 05902044 A1
[4] US 06154580 A1

### Bezugszeichenliste

- 100: Sensor-Anordnung
- 101: Sensor-Elektrode
- 102: Zeilen-Leitungen
- 103: Spalten-Leitungen
- 104: elektrische Verstärker-Einrichtungen
- 105: erster Matrix-Bereich
- 106: zweiter Matrix-Bereich
- 110: Verstärker-Element
- 111: Schalter-Element
- 112: Sensor-Fläche
- 200: Sensor-Anordnung
- 201a: erste Zeilen-Leitung
- 201b: zweite Zeilen-Leitung
- 201c: dritte Zeilen-Leitung
- 202a: erste Spalten-Leitung
- 202b: zweite Spalten-Leitung
- 202c: dritte Spalten-Leitung
- 203: Sensor-Feld
- 203a: aktiviertes Sensor-Feld
- 204: Kopplungs-Einrichtung
- 205: Sensor-Element
- 206: Mittel zum Erfassen von Summen-Stromflüssen
- 207: Dekodier-Einrichtung
- 300: Sensor-Anordnung
- 300a: Prinzipskizze
- 301: Zeilen-Leitung
- 302: Spalten-Leitung
- 303a: erster Teil-Bereich
- 303b: zweiter Teil-Bereich
- 303c: dritter Teil-Bereich
- 303d: vierter Teil-Bereich
- 304: Sensor-Felder
- 305: Verstärker-Einrichtung
- 400: Sensor-Feld
- 401: Zeilen-Leitung
- 402: Spalten-Leitung
- 403: Kopplungs-Einrichtung
- 404: Sensor-Element
- 410: Sensor-Feld
- 411: Zeilen-Leitung
- 412: Spalten-Leitung
- 413: Kopplungs-Einrichtung
- 414: Sensor-Element
- 500: Sensor-Feld
- 501: Zeilen-Leitung
- 502: Spalten-Leitung
- 503: Detektions-Transistor
- 504: Sensor-Element
- 510: Sensor-Feld
- 511: Zeilen-Leitung
- 512a: erste Spalten-Leitung
- 512b: zweite Spalten-Leitung
- 513: Detektions-Transistor
- 514: Sensor-Element
- 515: Kalibrier-Transistor
- 516: Kondensator
- 520: Sensor-Feld
- 521: Zeilen-Leitung
- 522a: erste Spalten-Leitung
- 522b: zweite Spalten-Leitung
- 523: Detektions-Transistor
- 524: Sensor-Element
- 525: Kalibrier-Transistor
- 526: Kondensator
- 527: Bipolar-Transistor
- 530: Sensor-Feld
- 531: Zeilen-Leitung
- 532a: erste Spalten-Leitung
- 532b: zweite Spalten-Leitung
- 533a: erster Detektions-Transistor
- 533b: zweiter Detektions-Transistor
- 534: Sensor-Element
- 600: erste Projektion
- 601: zweite Projektion
- 602: dritte Projektion
- 603: vierte Projektion
- 604: erstes Neuron
- 605: zweites Neuron
- 606: drittes Neuron
- 700: Sensor-Anordnung
- 701: Zeilen-Leitung
- 702: Spalten-Leitung
- 703: Sensor-Felder
- 704: Dekodier-Einrichtung
- 704a: erster Ausgang
- 704b: zweiter Ausgang
- 705: Verstärker-Einrichtung
- 800: Eingangs-Schnittstellen
- 801: Schalter
- 900: Trellis-Diagramm
- 901: Zeitachse
- 902: Trellis-Zustände zum Zeitpunkt t₀
- 903: Trellis-Zustände zum Zeitpunkt t₁
- 904: Pfeile
- 1000: Dekodier-Einrichtung
- 1001: Zeilendekodier-Einrichtung
- 1002: Spaltendekodier-Einrichtung
- 1003: Pfeile
- 1004: Ausgangssignale
- 1100: Eingänge
- 1101: Schalter
- 1102: Teil-Dekodier-Einrichtungen

## Patentansprüche

1. Sensor-Anordnung (100)
• mit einer Mehrzahl von in einer ersten Richtung angeordneten Zeilen-Leitungen (102);
• mit einer Mehrzahl von in mindestens einer zweiten Richtung angeordneten Spalten-Leitungen (103);
• mit einer Mehrzahl von in Kreuzungsbereichen von Zeilen-Leitungen (102) und Spalten-Leitungen (103) angeordneten Sensor-Feldern (203) mit
○ mindestens einer Kopplungs-Einrichtung (204) zum elektrischen Koppeln von jeweils einer Zeilen-Leitung (102) mit jeweils einer Spalten-Leitung (103);
○ einem Sensor-Element (205), das der mindestens einen Kopplungs-Einrichtung (204) zugeordnet ist, wobei das Sensor-Element (205) derart eingerichtet ist, dass das Sensor-Element (205) den elektrischen Stromfluss durch die mindestens eine zugeordnete Kopplungs-Einrichtung (204) beeinflusst;
• mit einer mit den Zeilen-Leitungen (102) und den Spalten-Leitungen (103) gekoppelten Dekodier-Einrichtung (207);
**dadurch gekennzeichnet,**
• **dass** die Sensor-Anordnung (100) ferner ein an einem jeweiligen End-Abschnitt von mindestens einem Teil der Zeilen-Leitungen (102) und von mindestens einem Teil der Spalten-Leitungen (103) elektrisch gekoppeltes Mittel (206) zum Erfassen eines jeweiligen Summen-Stromflusses aus den von den Sensor-Feldern (203) der jeweiligen Leitung bereitgestellten elektrischen Einzel-Stromflüssen aufweist;
• **dass** die Dekodier-Einrichtung (207) derart eingerichtet ist, dass sie mindestens einen Teil der elektrischen Summen-Stromflüsse, welche der Dekodier-Einrichtung (207) über die Zeilen-Leitungen (102) und die Spalten-Leitungen (103) zuführbar sind, auswertet und basierend darauf entscheidet, an welchen Sensor-Elementen (205) ein Sensor-Signal anliegen könnte.

2. Sensor-Anordnung (100) nach Anspruch 1,
• bei der die Dekodier-Einrichtung (207) in eine Zeilendekodier-Einrichtung (1001), der die elektrischen Summen-Stromflüsse der Zeilen-Leitungen (102) zuführbar sind, und in eine Spaltendekodier-Einrichtung (1002), der die elektrischen Summen-Stromflüsse der Spalten-Leitungen (103) zuführbar sind, aufgeteilt ist,
• wobei die Zeilendekodier-Einrichtung (1001) derart eingerichtet ist, dass aus mindestens einem Teil der elektrischen Summen-Stromflüsse der Zeilen-Leitungen (102) unabhängig von den Summen-Stromflüssen der Spalten-Leitungen (103) Informationen über diejenigen Sensor-Elemente (205) ermittelbar sind, an denen möglicherweise ein Sensor-Signal anliegt;
• wobei die Spaltendekodier-Einrichtung (1002) derart eingerichtet ist, dass aus mindestens einem Teil der elektrischen Summen-Stromflüsse der Spalten-Leitungen (103) unabhängig von den Summen-Stromflüssen der Zeilen-Leitungen (102) Informationen über diejenigen Sensor-Elemente (205) ermittelbar sind, an denen möglicherweise ein Sensor-Signal anliegt;
• wobei die Dekodier-Einrichtung (207) derart eingerichtet ist, dass mittels gemeinsamen Auswertens der von der Zeilendekodier-Einrichtung (1001) und der Spaltendekodier-Einrichtung (1002) ermittelten Informationen diejenigen Sensor-Elemente (205) ermittelbar sind, an denen ein Sensor-Signal anliegt.

3. Sensor-Anordnung (100) nach Anspruch 1,
bei der die Dekodier-Einrichtung (207) derart eingerichtet ist, dass das Ermitteln von denjenigen Sensor-Elementen (205), an denen ein Sensor-Signal anliegt, erfolgt, indem
• eine Fourier-Transformation der zeitabhängigen Summen-Stromflüsse der Zeilen-Leitungen (102) und der Spalten-Leitungen (103) durchgeführt wird;
• die Fourier-transformierten Summen-Stromflüsse der Zeilen-Leitungen (102) und der Spalten-Leitungen (103) paarweise miteinander multipliziert werden;
• mit den paarweise miteinander multiplizierten Summen-Stromflüssen eine Fourier-Rücktransformation durchgeführt wird.

4. Sensor-Anordnung (100) nach einem der Ansprüche 1 bis 3,
bei der die Dekodier-Einrichtung (207) derart eingerichtet ist, dass zum Ermitteln, ob an einem Sensor-Element (205) ein Sensor-Signal anliegt, mindestens ein Summen-Stromfluss mindestens einer nebenliegenden Zeilen-Leitung (102) und/oder mindestens einer nebenliegenden Spalten-Leitung (103) verwendet wird.

5. Sensor-Anordnung (100) nach einem der Ansprüche 1 bis 4,
bei der die Dekodier-Einrichtung (207) derart eingerichtet ist, dass zum Ermitteln, ob an einem Sensor-Element (205) ein Sensor-Signal anliegt, mindestens ein vorgegebenes zeitliches und/oder räumliches Referenzsignal verwendet wird.

6. Sensor-Anordnung (100) nach Anspruch 5,
bei der die Dekodier-Einrichtung (207) derart eingerichtet ist, dass zum Ermitteln, ob an einem Sensor-Element (205) ein Sensor-Signal anliegt, das mindestens eine vorgegebene zeitliche und/oder räumliche Referenzsignal an das erfasste Signal angepasst wird.

7. Sensor-Anordnung (100) nach Anspruch 5,
bei der die Dekodier-Einrichtung (207) derart eingerichtet ist, dass zum Ermitteln, ob an einem Sensor-Element (205) ein Sensor-Signal anliegt, mindestens zwei zeitliche und/oder räumliche Referenzsignale an das erfasste Signal angepasst werden.

8. Sensor-Anordnung (100) nach einem der Ansprüche 1 bis 7,
bei der die Dekodier-Einrichtung (207) derart eingerichtet ist, dass zum Ermitteln, ob an einem Sensor-Element (205) zu einem zweiten Zeitpunkt ein Sensor-Signal anliegt, eine vorgegebene Referenzinformation über Sensor-Signale zu einem ersten Zeitpunkt verwendet wird, welcher erste Zeitpunkt zeitlich vor dem zweiten Zeitpunkt liegt.

9. Sensor-Anordnung (100) nach einem der Ansprüche 1 bis 8,
bei der die Dekodier-Einrichtung (207) als Maximum-Likelihood Sequence Estimation-Dekoder oder als Maximum a posteriori-Dekoder ausgestaltet ist.

10. Sensor-Anordnung (100) nach einem der Ansprüche 1 bis 9, mit einer Spannungsquelle, die derart mit zumindest einem Teil der Zeilen-Leitungen (102) und der Spalten-Leitungen (103) gekoppelt ist, dass zumindest einem Teil der Kopplungs-Einrichtungen (204) eine vorgegebene Potentialdifferenz bereitgestellt ist.

11. Sensor-Anordnung (100) nach einem der Ansprüche 1 bis 10,
bei der mindestens eine Kopplungs-Einrichtung (204) eine von dem zugehörigen Sensor-Element (205) gesteuerte Stromquelle oder ein von dem zugehörigen Sensor-Element (205) gesteuerter Widerstand ist.

12. Sensor-Anordnung (100) nach einem der Ansprüche 1 bis 11,
bei der mindestens eine Kopplungs-Einrichtung (204) einen Detektions-Transistor (503) mit einem mit einer der Zeilen-Leitungen (102) gekoppelten ersten Source-/Drain-Anschluss, mit einem mit einer der Spalten-Leitungen (103) gekoppelten zweiten Source-/Drain-Anschluss und mit einem mit dem der Kopplungs-Einrichtung (204) zugeordneten Sensor-Element (205) gekoppelten Gate-Anschluss aufweist.

13. Sensor-Anordnung (100) nach einem der Ansprüche 1 bis 12,
bei der mindestens eine Kopplungs-Einrichtung (204) eine Kalibrier-Einrichtung zum Kalibrieren der Kopplungs-Einrichtung (204) aufweist.

14. Sensor-Anordnung (100) nach einem der Ansprüche 1 bis 13,
die derart eingerichtet ist, dass mindestens eine Kopplungs-Einrichtung (204) eine Deaktivierungsfunktion aufweist.

15. Sensor-Anordnung (100) nach den Ansprüchen 13 oder 14,
bei der die Kalibrier-Einrichtung einen Kalibrier-Transistor (515) mit einem mit der Zeilen-Leitung (102) gekoppelten ersten Source-/Drain-Anschluss, mit einem mit dem Gate-Anschluss des Detektions-Transistors (503) sowie mit einem mit dem zugeordneten Sensor-Element (205) gekoppelten Kondensator (516) gekoppelten zweiten Source-/Drain-Anschluss und mit einem mit einer weiteren Spalten-Leitung (103) gekoppelten Gate-Anschluss aufweist, wobei mittels der weiteren Spalten-Leitung (103) an den Gate-Anschluss des Kalibrier-Transistors (515) eine elektrische Kalibrier-Spannung anlegbar ist.

16. Sensor-Anordnung (100) nach einem der Ansprüche 1 bis 15,
bei der mindestens eine Kopplungs-Einrichtung (204) ein Verstärker-Element (305) zum Verstärken des elektrischen Einzel-Stromflusses der Kopplungs-Einrichtung (204) aufweist.

17. Sensor-Anordnung (100) nach den Ansprüchen 12 und 16,
bei der das Verstärker-Element (305) ein Bipolar-Transistor (527) mit einem mit der Zeilen-Leitung (102) gekoppelten Kollektor-Anschluss, einem mit der Spalten-Leitung gekoppelten Emitter-Anschluss und einem mit dem zweiten Source-/Drain-Anschluss des Detektions-Transistors (503) gekoppelten Basis-Anschluss aufweist.

18. Sensor-Anordnung (100) nach einem der Ansprüche 1 bis 17,
bei der zumindest ein Teil der Zeilen-Leitungen (102) und der Spalten-Leitungen (103) eine Verstärker-Einrichtung (305) zum Verstärken des in der jeweiligen Zeilen-Leitung (102) bzw. Spalten-Leitung (103) fließenden elektrischen Summen-Stromflusses aufweist.

19. Sensor-Anordnung (100) nach einem der Ansprüche 1 bis 18,
bei der zumindest ein Teil der Zeilen-Leitungen (102) und/oder der Spalten-Leitungen (103) eine Abtast-/Halte-Einrichtung zum Abspeichern des in der jeweiligen Zeilen-Leitung (102) bzw. Spalten-Leitung (103) fließende elektrischen Summen-Stromflusses zu einem vorgebbaren Zeitpunkt aufweist.

20. Sensor-Anordnung (100) nach einem der Ansprüche 1 bis 19,
bei der zumindest ein Sensor-Element (205) ein Ionensensitiver Feldeffekttransistor (ISFET) ist.

21. Sensor-Anordnung (100) nach einem der Ansprüche 1 bis 20,
bei der zumindest ein Sensor-Element (205) einen MOSFET aufweist.

22. Sensor-Anordnung (100) nach einem der Ansprüche 1 bis 21,
bei der zumindest ein Sensor-Element (205) ein auf elektromagnetische Strahlung empfindlicher Sensor ist.

23. Sensor-Anordnung (100) nach einem der Ansprüche 1 bis 22,
bei der die Sensor-Felder (203) im Wesentlichen rechteckförmig ausgebildet sind.

24. Sensor-Anordnung (100) nach Anspruch 23,
bei der die Zeilen-Leitungen (102) mit den Spalten-Leitungen (103) einen im Wesentlichen rechten Winkel einschließen.

25. Sensor-Anordnung (100) nach einem der Ansprüche 1 bis 22,
bei der die Sensor-Felder (203) im Wesentlichen wabenförmig ausgebildet sind.

26. Sensor-Anordnung (100) nach Anspruch 25,
bei der die Zeilen-Leitungen (102) mit den Spalten-Leitungen (103) einen Winkel von 60° einschließen und bei der unterschiedliche Spalten-Leitungen (103) entweder zueinander parallel sind oder miteinander einen Winkel von 60° einschließen.

27. Sensor-Anordnung (100) nach einem der Ansprüche 1 bis 26,
die in mindestens zwei voneinander unabhängig betreibbare Bereiche (105, 106) aufgeteilt ist, wobei die Sensor-Anordnung (100) derart eingerichtet ist, dass vorgebbar ist, welche der mindestens zwei Bereiche (105, 106) betrieben werden.

## Claims

1. Sensor arrangement (100)
• with a plurality of row lines (102) arranged in a first direction;
• with a plurality of column lines (103) arranged in at least a second direction;
• with a plurality of sensor areas (203) arranged in the crossover regions of the row lines (102) and the column lines (103) with
○ at least one coupling device (204) for electrically coupling of respective one row line (102) with respective one column line (103) ;
○ a sensor element (205) which is assigned to the at least one coupling device (204), wherein the sensor element (205) is configured such that the sensor element (205) influences the electric current flow through the at least one coupling device (204);
• with a decoding device (207) coupled to the row lines (102) and the column lines (103), **characterized in**
• **that** the sensor arrangement (100) further comprises a means (206) which is electrically coupled to a respective end section of at least a portion of the row lines (102) and of at least a portion of the column lines (103) for detecting a respective summation current flow from the individual electrical current flows provided by the sensor areas (203) of the respective lines;
• **that** the decoding device (207) is configured such that it evaluates at least a portion of the summation current flows which can be fed to the decoding device (207) via the row lines (102) and the column lines (103) and based on that decides at which sensor elements (205) a signal could be present.

2. Sensor arrangement (100) according to claim 1,
• in which the decoding device (207) is divided into a row decoding device (1001), to which the electrical summation current flows of the row lines (102) can be fed, and a column line decoding device (1002), to which the electrical summation current flows of the column lines (103) can be fed,
• wherein the row line decoding device (1001) is configured such that information about those sensor elements (205) at which a sensor signal is possibly present can be determined from at least a portion of the summation current flows of the row lines (102) independently of the summation current flows of the column lines (103);
• wherein the column line decoding device (1002) is configured such that information about those sensor elements (205) at which a sensor signal is possibly present can be determined from at least a portion of the summation current flows of the column lines (103) independently of the summation current flows of the row lines (102);
• wherein the decoding device (207) is configured such that those sensor elements (205) at which a sensor signal is present can be determined by means of joint evaluation of the information determined by the row decoding device (1001) and the column decoding device (1002).

3. Sensor-arrangement (100) according to claim 1,
in which the decoding device (207) is configured such that the determining of those sensor elements (205) at which a sensor signal is present is carried out by
• carrying out a Fourier transformation of the time-dependent summation current flows of the row lines (102) and of the column lines (103);
• multiplying the Fourier-transformed summation current flows of the row lines (102) and of the column lines (103) together in pairs;
• carrying out an inverse Fourier transformation with the summation current flows multiplied together in pairs.

4. Sensor arrangement (100) according to any of claims 1 to 3,
in which the decoding device (207) is configured such that in order to determine whether a sensor signal is present at a sensor element (205) at least one summation current flow of at least one adjacent row line (102) and/or of at least one adjacent column line (103) is used.

5. Sensor arrangement (100) according to any of claims 1 to 4,
in which the decoding device (207) is configured such that in order to determine whether a sensor signal is present at a sensor element (205) at least one predetermined temporal and/or spatial reference signal is used.

6. Sensor arrangement (100) according to claim 5,
in which the decoding device (207) is configured such that in order to determine whether a sensor signal is present at a sensor element (205) the at least one predetermined temporal and/or spatial reference signal is adapted to the detected signal.

7. Sensor arrangement (100) according to claim 5,
in which the decoding device (207) is configured such that in order to determine whether a sensor signal is present at a sensor element (205) at least two temporal and/or spatial reference signals are adapted to the detected signal.

8. Sensor arrangement (100) according to any of claims 1 to 7,
in which the decoding device (207) is configured such that in order to determine whether a sensor signal is present at a sensor element (205) at a second point in time a predetermined item of reference information about sensor signals at a first point in time is used, which first point in time temporally precedes the second point in time.

9. Sensor arrangement (100) according to any of claims 1 to 8,
in which the decoding device (207) is configured as a maximum likelihood sequence estimation decoder or as a maximum a posteriori decoder.

10. Sensor arrangement (100) according to any of claims 1 to 9,
with a voltage source which is coupled with at least a portion of the row lines (102) and of the column lines (103) such that a predetermined potential difference is provided for at least a portion of the coupling devices (204).

11. Sensor arrangement (100) according to any of claims 1 to 10,
in which at least one coupling device (204) is a current source controlled by the associated sensor element (205) or a resistor controlled by the associated sensor element (205).

12. Sensor arrangement (100) according to any of claims 1 to 11,
in which at least one coupling device (204) comprises a detection transistor (503) with a first source/drain terminal coupled to one of the row lines (102), with a second source/drain terminal coupled to one of the column lines (103) and with a gate terminal coupled to the sensor element (205) assigned to the coupling device (204).

13. Sensor arrangement (100) according to any of claims 1 to 12,
in which at least one coupling device (204) comprises a calibration device for calibrating the coupling device (204).

14. Sensor arrangement (100) according to any of claims 1 to 13,
which is configured such that at least one coupling device (204) comprises a deactivation function.

15. Sensor arrangement (100) according to the claims 13 or 14,
in which the calibration device comprises a calibration transistor (515) with a first source/drain terminal coupled to the row line (102), with a second source/drain terminal coupled to the gate terminal of the detection transistor (503) and also to a capacitor (516) coupled to the assigned sensor element (205) and with a gate transistor coupled to a further column line (103), wherein by means of the further column line (103) an electrical calibration voltage can be made present at the gate terminal of the calibration transistor (515).

16. Sensor arrangement (100) according to any of claims 1 to 15,
in which at least a coupling device (204) comprises an amplifier element (305) for amplifying the individual electrical current flows of the coupling device (204).

17. Sensor arrangement (100) according to the claims 12 and 16,
in which the amplifier element (305) comprises a bipolar transistor (527) with a collector terminal coupled with the row line (102), an emitter terminal coupled with the column line and a base terminal coupled to the second source/drain terminal of the detection transistor (503).

18. Sensor arrangement (100) according to any of claims 1 to 17,
in which at least a portion of the row lines (102) and of the column lines (103) comprise an amplifier device (305) for amplifying the electrical summation current flow flowing in the respective row line (102) and column line (103).

19. Sensor arrangement (100) according to any of claims 1 to 18,
in which at least a portion of the row lines (102) and/or of the column lines (103) comprise a sample/hold device for storing the summation current flow flowing in the respective row line (102) or in the respective column line (103) at a predeterminable point of time.

20. Sensor arrangement (100) according to any of claims 1 to 19,
in which at least one sensor element (205) is an ion-sensitive field-effect transistor (ISFET).

21. Sensor arrangement (100) according to any of claims 1 to 20,
in which at least one sensor element (205) comprises a MOSFET.

22. Sensor arrangement (100) according to any of claims 1 to 21,
in which at least one sensor element (205) is a sensor which is sensitive to electromagnetic radiation.

23. Sensor arrangement (100) according to any of claims 1 to 22,
in which the sensor areas (203) are formed essentially in a rectangular fashion.

24. Sensor arrangement (100) according to claim 23,
in which the row lines (102) enclose essentially a right angle with the column lines (103).

25. Sensor arrangement (100) according to any of claims 1 to 22,
in which the sensor areas (203) are formed essentially in a honeycomb-shaped fashion.

26. Sensor arrangement (100) according to claim 25,
in which the row lines (102) enclose an angle of 60° with the column lines (103) and in which different column lines (103) are either parallel or enclose an angle of 60° with one another.

27. Sensor arrangement (100) according to any of claims 1 to 26,
which is divided in at least two regions (105, 106) which can be operated independently, wherein the sensor arrangement (100) is configured such that it is predeterminable which of the at least two regions (105, 106) are operated.

## Revendications

1. Dispositif (100) de capteur
• comprenant une multiplicité de conducteurs (102) de ligne disposés dans une première direction ;
• comprenant une multiplicité de conducteurs (103) de colonne disposés dans au moins une deuxième direction ;
• comprenant une multiplicité de champs (203) de capteur disposés dans des zones d'entrecroisement de conducteurs (102) de ligne et de conducteurs (103) de colonne et comprenant
○ au moins un dispositif (204) de couplage, pour coupler électriquement respectivement un conducteur (102) de ligne à respectivement un conducteur (103) de colonne ;
○ un élément (205) de capteur, qui est associé à au moins un dispositif (204) de couplage, l'élément (205) de capteur étant tel que l'élément (205) de capteur influence le flux de courant électrique passant dans le au moins un dispositif (204) de couplage associé ;
• comprenant un dispositif (207) de décodage couplé aux conducteurs (102) de ligne et aux conducteurs (103) de colonne ;
**caractérisé**
• **en ce que** le dispositif (100) de capteur comporte, en outre, un moyen (206), qui est couplé électriquement à une section d'extrémité respective d'au moins une partie des conducteurs (102) de ligne et d'au moins une partie des conducteurs (103) de colonne et qui est destiné à détecter un flux de courant somme respectif composé des flux de courant électriques individuels procurés par les champs (203) de capteurs du conducteur respectif ;
• **en ce que** le dispositif (207) de décodage est tel qu'il évalue au moins une partie des flux de courant somme électriques, qui peuvent être envoyés au dispositif (207) de décodage par les conducteurs (102) de ligne et par les conducteurs (103) de colonne, et décide sur cette base sur quel élément (205) de capteur un signal de capteur pourrait s'appliquer.

2. Dispositif (100) de capteur suivant la revendication 1,
• dans lequel le dispositif (207) de décodage est subdivisé en un dispositif (1001) de décodage de ligne, auquel peuvent être envoyés des flux de courant somme électriques des conducteurs (102) de ligne, et en un dispositif (1002) de décodage de colonne, auquel peuvent être envoyés des flux de courant somme électriques des conducteurs (103) de colonne ;
• dans lequel le dispositif (1001) de décodage de ligne est tel qu'à partir d'au moins une partie des flux de courant somme électriques des conducteurs (102) de ligne et indépendamment des flux de courant somme des conducteurs (103) de colonne, des informations peuvent être déterminées sur les éléments (205) de capteur, auxquels s'applique éventuellement un signal de capteur ;
• dans lequel le dispositif (1002) de décodage de colonne est tel qu'à partir d'au moins une partie des flux de courant somme électriques des conducteurs (103) de colonne et indépendamment des flux de courant somme des conducteurs (102) de ligne, des informations peuvent être déterminées sur les éléments (205) de capteur, auxquels s'applique éventuellement un signal de capteur ;
• dans lequel le dispositif (207) de décodage est tel qu'au moyen d'une évaluation commune des informations déterminées par le dispositif (1001) de décodage de ligne et le dispositif (1002) de décodage de colonne, les éléments (205) de capteur auxquels s'applique un signal de capteur peuvent être déterminés.

3. Dispositif (100) de capteur suivant la revendication 1, dans lequel le dispositif (207) de décodage est tel que la détermination des éléments (205) de capteur auxquels s'applique un signal de capteur s'effectue en
• effectuant une transformation de Fourier des flux de courant somme en fonction du temps des conducteurs (102) de ligne et des conducteurs (103) de colonne ;
• multipliant les flux de courant somme à transformation de Fourier des conducteurs (102) de ligne et des conducteurs (103) de colonne par paire entre eux ;
• effectuant une retransformation de Fourier avec les flux de courant somme multipliés entre eux par paire.

4. Dispositif (100) de capteur suivant l'une des revendications 1 à 3,
dans lequel le dispositif (207) de décodage est tel que, pour déterminer si un signal de capteur s'applique à un élément (205) de capteur, il est utilisé au moins un flux de courant somme d'au moins un conducteur (102) de ligne voisin et/ou d'au moins un conducteur (103) de colonne voisin.

5. Dispositif (100) de capteur suivant l'une des revendications 1 à 4,
dans lequel le dispositif (207) de décodage est tel que, pour déterminer si un signal de capteur s'applique à un élément (205) de capteur, il est utilisé au moins un signal de référence temporel et/ou spatial prescrit.

6. Dispositif (100) de capteur suivant la revendication 5,
dans lequel le dispositif (207) de décodage est tel que, pour déterminer si un signal de capteur s'applique à un élément (205) de capteur, le au moins un signal de référence temporel et/ou spatial prescrit est adapté au signal détecté.

7. Dispositif (100) de capteur suivant la revendication 5,
dans lequel le dispositif (207) de décodage est tel que, pour déterminer si un signal de capteur s'applique à un élément (205) de capteur, au moins deux signaux de référence temporels et/ou spatiaux sont adaptés au signal détecté.

8. Dispositif (100) de capteur suivant l'une des revendications 1 à 7,
dans lequel le dispositif (207) de décodage est tel que, pour déterminer si un signal de capteur s'applique à un élément (205) de capteur à un deuxième instant, il est utilisé une information de référence prescrite sur des signaux de capteur à un premier instant, lequel premier instant précède dans le temps le deuxième instant.

9. Dispositif (100) de capteur suivant l'une des revendications 1 à 8,
dans lequel le dispositif (207) de décodage est conformé en décodeur de maximum-likelihood sequence estimation ou en décodeur de maximum a posteriori.

10. Dispositif (100) de capteur suivant l'une des revendications 1 à 9,
comprenant une source de tension, qui est couplée à au moins une partie des conducteurs (102) de ligne et des conducteurs (103) de colonne, de manière à procurer une différence de potentiel prescrit à au moins une partie des dispositifs (204) de couplage.

11. Dispositif (100) de capteur suivant l'une des revendications 1 à 10,
dans lequel au moins un dispositif (204) de couplage est une source de courant commandée par l'élément (205) de capteur associé ou une résistance commandée par l'élément (205) de capteur associé.

12. Dispositif (100) de capteur suivant l'une des revendications 1 à 11,
dans lequel au moins un dispositif (204) de couplage comporte un transistor (503) de détection ayant une première borne de source/drain couplée à l'un des conducteurs (102) de ligne, une deuxième borne de source/drain couplée à l'un de conducteurs (103) de colonne et une borne de grille couplée à un élément (205) de capteur associé au dispositif (204) de couplage.

13. Dispositif (100) de capteur suivant l'une des revendications 1 à 12,
dans lequel au moins un dispositif (204) de couplage a un dispositif d'étalonnage pour étalonner le dispositif (204) de couplage.

14. Dispositif (100) de capteur suivant l'une des revendications 1 à 13,
qui est tel qu'au moins un dispositif (204) de couplage comporte une fonction de désactivation.

15. Dispositif (100) de capteur suivant les revendications 13 ou 14,
le dispositif d'étalonnage comporte un transistor (515) d'étalonnage ayant une première borne de source/drain couplée au conducteur (102) de ligne, une deuxième borne de source/drain couplée à la borne de grille du transistor (503) de détection ainsi qu'à un condensateur (516) couplé à l'élément (205) du détecteur associé et une borne de grille couplée à un autre conducteur (103) de colonne, dans lequel, au moyen de l'autre conducteur (103) de colonne, une tension électrique d'étalonnage peut être appliquée à la borne de grille du transistor (515) d'étalonnage.

16. Dispositif (100) de capteur suivant l'une des revendications 1 à 15,
dans lequel au moins un dispositif (204) de couplage comporte un élément (305) amplificateur pour amplifier le courant de flux de courant individuel électrique du dispositif (204) de couplage.

17. Dispositif (100) de capteur suivant les revendications 12 et 16,
dans lequel l'élément (305) amplificateur comporte un transistor (527) bipolaire ayant une borne de collecteur couplée au conducteur (102) de ligne, une borne d'émetteur couplée au conducteur de colonne et une borne de base couplée à la deuxième borne de source/drain du transistor (503) de détection.

18. Dispositif (100) de capteur suivant l'une des revendications 1 à 17,
dans lequel au moins une partie des conducteurs (102) de ligne et des conducteurs (103) de colonne comporte des dispositifs (305) amplificateurs, pour amplifier le flux de courant somme électrique passant dans le conducteur (102) de ligne respectif ou le conducteur (103) de colonne respectif.

19. Dispositif (100) de capteur suivant l'une des revendications 1 à 18,
dans lequel au moins une partie des conducteurs (102) de ligne et/ou des conducteurs (103) de colonne comporte un dispositif d'interrogation/maintien, pour mémoriser le flux de courant somme électrique passant dans le conducteur (102) de ligne respectif ou le conducteur (103) de colonne respectif à un instant pouvant être prescrit.

20. Dispositif (100) de capteur suivant l'une des revendications 1 à 19,
dans lequel au moins un élément (205) de capteur est un transistor à effet de champ sensible aux ions (ISFET).

21. Dispositif (100) de capteur suivant l'une des revendications 1 à 20,
dans lequel au moins un élément (205) de capteur comporte un MOSFET.

22. Dispositif (100) de capteur suivant l'une des revendications 1 à 21,
dans lequel au moins un élément (205) de capteur est un capteur sensible au rayonnement électromagnétique.

23. Dispositif (100) de capteur suivant l'une des revendications 1 à 22,
dans lequel les champs (203) de capteur sont sensiblement rectangulaires.

24. Dispositif (100) de capteur suivant la revendication 23, les conducteurs (102) de ligne font sensiblement un angle droit avec les conducteurs (103) de colonne.

25. Dispositif (100) de capteur suivant l'une des revendications 1 à 22,
dans lequel les champs (203) de capteur sont sensiblement en nids d'abeilles.

26. Dispositif (100) de capteur suivant la revendication 25,
dans lequel les conducteurs (102) de ligne font un angle de 60° avec les conducteurs (103) de colonne et dans lequel des conducteurs (103) de colonne différents sont parallèles entre eux ou font entre eux un angle de 60°.

27. Dispositif (100) de capteur suivant l'une des revendications 1 à 26,
qui est subdivisé en au moins deux zones (105, 106) pouvant fonctionner indépendamment l'une de l'autre, le dispositif (100) de capteur étant tel que l'on peut prescrire celle des au moins deux zones (105, 106) qui fonctionne.
